(19) Europäisches Patentamt
European Patent Office
Office européen des brevets

(11) EP 2 291 522 B1

(12) EUROPEAN PATENT SPECIFICATION

(45) Date of publication and mention of the grant of the patent:
**04.12.2013 Bulletin 2013/49**

(21) Application number: **09749332.4**

(22) Date of filing: **19.05.2009**

(51) Int Cl.:
***C12N 15/12*** *(2006.01)*
***C12Q 1/68*** *(2006.01)*
***C12N 5/22*** *(2006.01)*
***C07K 14/705*** *(2006.01)*
***C12N 5/16*** *(2006.01)*
***A61P 9/00*** *(2006.01)*
***G01N 33/50*** *(2006.01)*
***C12N 15/63*** *(2006.01)*

(86) International application number:
**PCT/AU2009/000624**

(87) International publication number:
**WO 2009/140724 (26.11.2009 Gazette 2009/48)**

(54) **HERG MUTANTS AND USES THEREOF**

HERG-MUTANTEN UND DEREN VERWENDUNGEN

MUTANTS DE HERG ET LEURS UTILISATIONS

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **20.05.2008 AU 2008902492**

(43) Date of publication of application:
**09.03.2011 Bulletin 2011/10**

(73) Proprietor: **Victor Chang Cardiac Research Institute Limited**
**New South Wales 2010 (AU)**

(72) Inventors:
- **VANDENBERG, James Ian**
  **Willoughby, NSW 2068 (AU)**
- **PERRIN, Mark Jonathan**
  **Ottawa ON**
  **K1Z 7G4 (CA)**

(74) Representative: **Stephen, Robert John et al**
**Olswang LLP**
**90 High Holborn**
**London WC1V 6XX (GB)**

(56) References cited:
**WO-A1-00/06772 WO-A2-2005/042732**

- **LAITINEN P ET AL: "Survey of the coding region of the HERG gene in long QT syndrome reveals six novel mutations and an amino acid polymorphism with possible phenotypic effects.", HUMAN MUTATION JUN 2000 LNKD-PUBMED:10862094, vol. 15, no. 6, June 2000 (2000-06), pages 580-581, XP000002656222, ISSN: 1098-1004**
- **CLARKE, C.E ET AL.: 'Effect of S5P alpha-helix charge mutants on inactivation of hERG K+ channels' JOURNAL OF PHYSIOLOGY vol. 573, no. 2, 2006, pages 291 - 304, XP008132405**
- **CORDEIRO, J.M. ET AL.: 'Modulation of IKr inactivation by mutation N588K in KCNH2: A link to arrhythmogenesis in short QT syndrome' CARDIOVASCULAR RESEARCH vol. 67, 2005, pages 498 - 509, XP004985953**
- **PERRIN, M.J. ET AL.: 'Drug binding to the Inactivated State Is Necessary but Not Sufficient for High-Affinity Binding to Human Ether-a-go-go-Related Gene Channels' MOLECULAR PHARMACOLOGY vol. 74, no. 5, 2008, pages 1443 - 1452, XP008132398**
- **PERRIN, M.J. ET AL.: 'Human ether-a-go-go related gene (hERG) K+ channels: Function and dysfunction' PROGRESS IN BIOPHYSICS AND MOLECULAR BIOLOGY vol. 98, 2008, pages 137 - 148, XP026586005**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 291 522 B1

SEQUENCE LISTING

<110> Victor Chang Cardiac Research Institute Limited
<120> hERG mutants and uses thereof
<130> 206143380
<160> 10
<170> PatentIn version 3.4
<210> 1
<211> 3486
<212> DNA
<213> human
<220>
<221> misc_feature
<222> (1572)..(1572)
<223> n is a, c, g, or t
<220>
<221> misc_feature
<222> (1596)..(1596)
<223> n is a, c, g, or t
<220>
<221> misc_feature
<222> (1605)..(1605)
<223> n is a, c, g, or t
<220>
<221> misc_feature
<222> (1608)..(1608)
<223> n is a, c, g, or t
<220>
<221> misc_feature
<222> (1752)..(1752)
<223> n is a, c, g, or t
<220>
<221> misc_feature
<222> (1761)..(1761)
<223> n is a, c, g, or t
<400> 1

```
atgccggtgc ggaggggcca cgtcgcgccg cagaacacct tcctggacac catcatccgc     60
aagtttgagg gccagagccg taagttcatc atcgccaacg ctcgggtgga gaactgcgcc    120
gtcatctact gcaacgacgg cttctgcgag ctgtgcggct actcgcgggc cgaggtgatg    180
cagcgaccct gcacctgcga cttcctgcac gggccgcgca cgcagcgccg cgctgccgcg    240
cagatcgcgc aggcactgct gggcgccgag gagcgcaaag tggaaatcgc cttctaccgg    300
aaagatggga gctgcttcct atgtctggtg gatgtggtgc ccgtgaagaa cgaggatggg    360
gctgtcatca tgttcatcct caatttcgaa gtggtgatgg agaaggacat ggtggggtcc    420
ccggctcatg acaccaacca ccggggcccc cccaccagct ggctggcccc aggccgcgcc    480
aagaccttcc gcctgaagct gcccgcgctg ctggcgctga cggcccggga gtcgtcggtg    540
cggtcgggcg gcgcgggcgg cgcgggcgcc ccgggggccg tggtggtgga cgtggacctg    600
acgcccgcgg cacccagcag cgagtcgctg gccctggacg aagtgacagc catggacaac    660
cacgtggcag ggctcgggcc cgcggaggag cggcgtgcgc tggtgggtcc cggctctccg    720
ccccgctcag cgcccggcca gctcccatcg ccccgggcgc acagcctcaa ccccgacgcc    780
tcgggctcca gctgcagcct ggcccggacg cgctcccgag aaagctgcgc cagcgtgcgc    840
cgcgcctcgt cggccgacga catcgaggcc atgcgcgccg gggtgctgcc cccgccaccg    900
cgccacgcca gcaccggggc catgcaccca ctgcgcagcg gcttgctcaa ctccacctcg    960
gactccgacc tcgtgcgcta ccgcaccatt agcaagattc cccaaatcac cctcaacttt   1020
gtggacctca agggcgaccc cttcttggct tcgcccacca gtgaccgtga gatcatagca   1080
cctaagataa aggagcgaac ccacaatgtc actgagaagg tcacccaggt cctgtccctg   1140
ggcgccgacg tgctgcctga gtacaagctg caggcaccgc gcatccaccg ctggaccatc   1200
ctgcattaca gccccttcaa ggccgtgtgg gactggctca tcctgctgct ggtcatctac   1260
acggctgtct tcacacccta ctcggctgcc ttcctgctta aggagacgga agaaggcccg   1320
ccggcgaccg agtgtggcta tgcatgccag ccgctggctg tggtggacct catcgtggac   1380
atcatgttca ttgtggacat cctcatcaac ttccgcacca cctacgtcaa tgccaacgag   1440
gaggtggtca gccaccccgg ccgcatcgcc gtccactact tcaagggctg gttcctcatc   1500
gacatggtgg ccgccatccc cttcgacctg ctcatcttcg gctctggctc tgaggagctg   1560
atcgggctgy knaagacggc gcgcctgctg cggyyngtgc gcgyngyncg gmarctggat   1620
cgctactcag agtacggcgc ggccgtgctg ttcttgctca tgtgtacctt tgcgctcatc   1680
gcgcactggc tagcctgcat ctggtacgca attggcaaca tggagcagcc acacatggac   1740
tcacgcatcd snttggctgma nrayctgggc gaccagatag gcaaacccta caacagctca   1800
ggcctgggcg gcccctccat caaggacaag tatgtgacgg cgctctactt caccttcagc   1860
```

agcctcacca gtgtgggctt cggcaacgtc tctcccaaca ccaactcaga gaagatcttc 1920
tccatctgcg tcatgctcat tggctccctc atgtatgcta gcatcttcgg caacgtgtcg 1980
gccatcatcc agcggctgta ctcgggcaca gcccgctacc acacacagat gctgcgggtg 2040
cgggagttca tccgcttcca ccagatcccc aatcccctgc gccagcgcct cgaggagtac 2100
ttccagcacg cctggtccta caccaacggc atcgacatga acgcggtgct gaagggcttc 2160
cctgagtgcc tgcaggctga catctgcctg cacctgaacc ggtcactgct gcagcactgc 2220
aaacccttcc gaggggccac caagggctgc cttcgggccc tggccatgaa gttcaagacc 2280
acacatgcac cgccagggga cacactggtg catgctgggg acctgctcac cgccctgtac 2340
ttcatctccc ggggctccat cgagatcctg cggggcgacg tcgtcgtggc catcctgggg 2400
aagaatgaca tctttgggga gcctctgaac ctgtatgcaa ggcctggcaa gtcgaacggg 2460
gatgtgcggg ccctcaccta ctgtgaccta cacaagatcc atcgggacga cctgctggag 2520
gtgctggaca tgtaccctga gttctctgat cacttctggt ccagcctgga gatcaccttc 2580
aacctgcgag ataccaacat gatcccgggc tcccccggca gtacggagtt agagggtggc 2640
ttcagtcggc aacgcaagcg caagttgtcc ttccgcaggc gcacggacaa ggacacggag 2700
cagccagggg aggtgtcggc cttggggccg ggccgggcgg gggcagggcc gagtagccgg 2760
ggccggccgg gggggccgtg gggggagagc ccgtccagtg gcccctccag ccctgagagc 2820
agtgaggatg agggcccagg ccgcagctcc agcccctcc gcctggtgcc cttctccagc 2880
cccaggcccc ccggagagcc gccgggtggg gagcccctga tggaggactg cgagaagagc 2940
agcgacactt gcaacccct gtcaggcgcc ttctcaggag tgtccaacat tttcagcttc 3000
tggggggaca gtcggggccg ccagtaccag gagctccctc gatgccccgc ccccaccccc 3060
agcctcctca acatccccct ctccagcccg ggtcggcggc ccggggcga cgtggagagc 3120
aggctggatg ccctccagcg ccagctcaac aggctggaga cccggctgag tgcagacatg 3180
gccactgtcc tgcagctgct acagaggcag atgacgctgg tcccgcccgc ctacagtgct 3240
gtgaccaccc cggggcctgg ccccacttcc acatccccgc tgttgcccgt cagcccctc 3300
cccaccctca ccttggactc gctttctcag gtttcccagt tcatggcgtg tgaggagctg 3360
cccccggggg ccccagagct tccccaagaa ggccccacac gacgcctctc cctaccgggc 3420
cagctggggg ccctcacctc ccagcccctg cacagacacg gctcggaccc gggcagtacg 3480
cgttag 3486

<210> 2
<211> 21
<212> DNA
<213> human
<400> 2
cttctcctac tgttattgtg g 21
<210> 3
<211> 30
<212> DNA
<213> human

<400> 3
cttctcctac tgttattgcc tcccccaccg 30
<210> 4
<211> 24
<212> DNA
<213> human
<400> 4
gttgtcgtag tggctgccgc agcg 24
<210> 5
<211> 24
<212> DNA
<213> human
<400> 5
gttgtcgtag tggctgccgc agcg 24
<210> 6
<211> 12
<212> DNA
<213> human
<400> 6
aaaaagcaac ag 12
<210> 7
<211> 30
<212> DNA
<213> human
<400> 7
ggtggcggag ggtcttcctc atcgagtagc 30
<210> 8
<211> 12
<212> DNA
<213> human
<400> 8
catcacaaaa ag 12
<210> 9
<211> 12
<212> DNA
<213> human
<400> 9
aataacgatg ac 12
<210> 10
<211> 3480
<212> DNA
<213> human
<400> 10

```
atgccggtgc ggaggggcca cgtcgcgccg cagaacacct tcctggacac catcatccgc    60
aagtttgagg gccagagccg taagttcatc atcgccaacg ctcgggtgga gaactgcgcc   120
gtcatctact gcaacgacgg cttctgcgag ctgtgcggct actcgcgggc cgaggtgatg   180
cagcgaccct gcacctgcga cttcctgcac gggccgcgca cgcagcgccg cgctgccgcg   240
cagatcgcgc aggcactgct gggcgccgag gagcgcaaag tggaaatcgc cttctaccgg   300
aaagatggga gctgcttcct atgtctggtg gatgtggtgc ccgtgaagaa cgaggatggg   360
gctgtcatca tgttcatcct caatttcgag gtggtgatgg agaaggacat ggtggggtcc   420
ccggctcatg acaccaacca ccggggcccc cccaccagct ggctggcccc aggccgcgcc   480
aagaccttcc gcctgaagct gcccgcgctg ctggcgctga cggcccggga gtcgtcggtg   540
cggtcgggcg gcgcgggcgg cgcgggcgcc ccgggggccg tggtggtgga cgtggacctg   600
acgcccgcgg cacccagcag cgagtcgctg gccctggacg aagtgacagc catggacaac   660
cacgtggcag ggctcgggcc cgcggaggag cggcgtgcgc tggtgggtcc cggctctccg   720
ccccgcagcg cgcccggcca gctcccatcg ccccgggcgc acagcctcaa ccccgacgcc   780
tcgggctcca gctgcagcct ggcccggacg cgctcccgag aaagctgcgc cagcgtgcgc   840
cgcgcctcgt cggccgacga catcgaggcc atgcgcgccg gggtgctgcc cccgccaccg   900
cgccacgcca gcaccggggc catgcaccca ctgcgcagcg gcttgctcaa ctccacctcg   960
```

gactccgacc tcgtgcgcta ccgcaccatt agcaagattc cccaaatcac cctcaacttt 1020
gtggacctca agggcgaccc cttcttggct tcgcccacca gtgaccgtga gatcatagca 1080
cctaagataa aggagcgaac ccacaatgtc actgagaagg tcacccaggt cctgtccctg 1140
ggcgccgacg tgctgcctga gtacaagctg caggcaccgc gcatccaccg ctggaccatc 1200
ctgcattaca gccccttcaa ggccgtgtgg gactggctca tcctgctgct ggtcatctac 1260
acggctgtct tcacacccta ctcggctgcc ttcctgctga aggagacgga agaaggcccg 1320
cctgctaccg agtgtggcta cgcctgccag ccgctggctg tggtggacct catcgtggac 1380
atcatgttca ttgtggacat cctcatcaac ttccgcacca cctacgtcaa tgccaacgag 1440
gaggtggtca gccaccccgg ccgcatcgcc gtccactact tcaagggctg gttcctcatc 1500
gacatggtgg ccgccatccc cttcgacctg ctcatcttcg gctctggctc tgaggagctg 1560
atcgggctgc tgaagactgc gcggctgctg cggctggtgc gcgtggcgcg gaagctggat 1620
cgctactcag agtacggcgc ggccgtgctg ttcttgctca tgtgcacctt tgcgctcatc 1680
gcgcactggc tagcctgcat ctggtacgcc atcggcaaca tggagcagcc acacatggac 1740
tcacgcatcg gctggctgca caacctgggc gaccagatag gcaaacccta caacagcagc 1800
ggcctgggcg gcccctccat caaggacaag tatgtgacgg cgctctactt caccttcagc 1860
agcctcacca gtgtgggctt cggcaacgtc tctcccaaca ccaactcaga gaagatcttc 1920
tccatctgcg tcatgctcat tggctccctc atgtatgcta gcatcttcgg caacgtgtcg 1980
gccatcatcc agcggctgta ctcgggcaca gcccgctacc acacacagat gctgcgggtg 2040
cgggagttca tccgcttcca ccagatcccc aatcccctgc gccagcgcct cgaggagtac 2100
ttccagcacg cctggtccta caccaacggc atcgacatga acgcggtgct gaagggcttc 2160
cctgagtgcc tgcaggctga catctgcctg cacctgaacc gctcactgct gcagcactgc 2220
aaacccttcc gaggggccac caagggctgc cttcgggccc tggccatgaa gttcaagacc 2280
acacatgcac cgccagggga cacactggtg catgctgggg acctgctcac cgccctgtac 2340
ttcatctccc ggggctccat cgagatcctg cggggcgacg tcgtcgtggc catcctgggg 2400
aagaatgaca tctttgggga gcctctgaac ctgtatgcaa ggcctggcaa gtcgaacggg 2460
gatgtgcggg ccctcaccta ctgtgaccta cacaagatcc atcgggacga cctgctggag 2520
gtgctggaca tgtaccctga gttctccgac cacttctggt ccagcctgga gatcaccttc 2580
aacctgcgag ataccaacat gatcccgggc tcccccggca gtacggagtt agagggtggc 2640
ttcagtcggc aacgcaagcg caagttgtcc ttccgcaggc gcacggacaa ggacacggag 2700
cagccagggg aggtgtcggc cttggggccg ggccgggcgg gggcagggcc gagtagccgg 2760
ggccggccgg gggggccgtg gggggagagc ccgtccagtg gcccctccag ccctgagagc 2820
agtgaggatg agggcccagg ccgcagctcc agccccctcc gcctggtgcc cttctccagc 2880
cccaggcccc ccggagagcc gccgggtggg gagcccctga tggaggactg cgagaagagc 2940
agcgacactt gcaacccccct gtcaggcgcc ttctcaggag tgtccaacat tttcagcttc 3000
tgggggggaca gtcggggccg ccagtaccag gagctccctc gatgccccgc ccccaccccc 3060

```
agcctcctca acatccccct ctccagcccg ggtcggcggc cccggggcga cgtggagagc   3120
aggctggatg ccctccagcg ccagctcaac aggctggaga cccggctgag tgcagacatg   3180
gccactgtcc tgcagctgct acagaggcag atgacgctgg tcccgcccgc ctacagtgct   3240
gtgaccaccc cggggcctgg ccccacttcc acatccccgc tgttgcccgt cagcccccctc  3300
cccaccctca ccttggactc gctttctcag gtttcccagt tcatggcgtg tgaggagctg   3360
cccccggggg ccccagagct tccccaagaa ggccccacac gacgcctctc cctaccgggc   3420
cagctggggg ccctcacctc ccagcccctg cacagacacg gctcggaccc gggcagttag   3480
```

## Description

Technical Field

The present invention relates to human ether-à-go-go related gene (hERG) mutants and uses thereof for screening potential drug binding to hERG protein.

Background Art

The human ether-à-go-go related gene (hERG) encodes the pore-forming subunit of the ion channel that conducts the rapid component of the delayed rectifier potassium current ($I_{Kr}$) in the heart. Gain and loss of function mutations in hERG may result in the clinical conditions of Short QT syndrome and Long QT syndrome respectively, underscoring the crucial role of hERG in maintaining electrical stability in the heart. The congenital form of LQTS is uncommon and may result from mutations in ten different genes - most encoding ion channels or their regulatory subunits. Acquired LQTS, through drug-induced blockade of hERG, is more frequent and the most common reason for the withdrawal of medications from the pharmaceutical market. Consequently, early assessment of a drug's affinity for hERG has been mandated, and obliges the need for a detailed understanding of how drugs bind to hERG.

It is well established that drugs bind in the central cavity of the pore region of hERG and that channels need to open before this can occur. At depolarized potentials, hERG channels can exist in either an open or an inactivated state, yet it has not been established whether the open or the inactivated state is the preferred drug binding state. Evidence in support of state-dependent drug binding primarily comes from studies showing reduced affinity for mutant channels that either abolish (S620T; G628C+S631 C) or reduce inactivation (S631A). These mutations, however, lie proximate to the selectivity filter and putative drug-binding pocket (see Figure 1) and so may affect drug-block directly or indirectly through local changes in the drug binding pocket. To address this concern the present inventors have investigated whether mutants to residues remote from the central pore that affect inactivation would affect drug binding to hERG in a manner similar to that reported for the S631A and S620T mutants.

The present inventors have now generated new mutants of hERG useful in screening for drug affinity for hERG.

Disclosure of Invention

The invention relates to an array for screening potential drug binding to hERG protein and to a stable mammalian cell line containing an expression vector as described in claims 1 to 12. An isolated nucleic acid molecule capable of expressing an human ether-à-go-go related gene (hERG) mutant that enhances hERG inactivation, wherein the mutation is not located in the vicinity of the drug-binding pocket within the cavity of the ion conduction pore of the hERG protein is also described.

The mutation may be located on the alpha-helix of the S5P linker. The mutation may be N588E, H587K, G584S, or Q592D. It will be appreciated, however, that other mutations not located in the drug-binding pocket of the hERG protein, but which still enhance inactivation, would also be suitable.

The present inventors have mutated residues N588, H587, G584 and Q592, located on the alpha-helix of the S5P linker, which is distant to the drug binding pocket, to either glutamate (N588E), lysine (N587K), serine (G584S) or aspartate (Q592D). Such mutations affect inactivation in hERG protein activity when expressed in a cell. Other candidate regions include all regions that are not part of the drug binding pocket. This includes the outer pore helix (e.g. F557A), voltage-sensor domain (e.g. A527V) both the N-terminal and C-terminal cytoplasmic domains, and the intracellular and extracellular linkers that join the transmembrane helical segments.

The isolated nucleic acid molecule may encode an hERG mutant containing one or more mutations substantially as set out in Figure 12 (SEQ ID NO: 1).

The nucleic acid molecule may contain one or more additional mutations such as L524W, L532P, V535A, A536V, K538Q that promote activation of the ion channel.

Also described is a vector for expressing an isolated nucleic acid molecule described herein.

Preferably, the vector contains a promoter suitable for expression, preferably high level expression, in mammalian cells and an antibiotic resistance gene to aid selection of cells expressing the hERG channel.

In one aspect, the present invention provides a stable mammalial cell line containing an expression vector containing an isolated nucleic acid molecule expressing a hERG mutant containing the N588E mutation as set out in SEQ ID No. 9 that enhances hERG inactivation in a cell.

Preferably, the cell is selected from, but not limited to, Chinese hamster ovary cells, human embryonic kidney cells or transformed African Green Monkey kidney fibroblast cells

Stable cell lines can be made using techniques known to the art and included antibiotic selection followed by clonal expansion.

The cell line is particularly suitable for screening potential drug binding to hERG protein.

In one aspect, the present invention provides an assay for screening potential drug binding to hERG protein comprising:

providing a stable mammalian cell line containing a nucleic acid molecule expressing a hERG N588E mutant that enhances hERG inactivation of the cell; incubating a test drug with the stable cell lines and
determining activity of the hERG mutant protein in the effects onion flux or voltage in the stable mammalian cell line.

The assay may involve either ion flux or voltage changes then using patch clamp assays, Rb+ efflux assays as a surrogate for K+ flux or fluorescence to assay voltage changes. Drugs will be incubated with cells and the effects on ion flux or voltage monitored. Assay conditions may include presence of high Rb+ or high K+ (to enhance entry into inactivated state) - then ion flux or voltage assayed following removal of the high K+ or Rb+. In some circumstances, the incorporation of mutations that enhance activation of hERG channels may obviate the need for high Rb+ or high K+ conditions.

Also described is the use of the isolated nucleic acid molecule described above or use of the cell line described above in an assay for screening potential drug binding to hERG protein.

The present inventors have characterised the binding of four high-affinity blockers (dofetilide, cisapride, astemizole, and terfenadine) as well as four low-affinity blockers (quinidine, perhexiline, erythromycin and dl-sotalol). All four high affinity blockers exhibited reduced affinity for the inactivation deficient mutants (N588K and S620T) whereas only dl-sotalol among the low affinity blockers showed reduced affinity for N588K. In all cases where binding was affected by inactivation deficient mutants, the affinity for S620T was markedly lower than for N588K mutant channels. A kinetic model of drug binding indicated that the difference between drug binding to wild type (WT), N588K and S620T channels can be explained by the kinetics of drug binding with the affinity for the open state being reduced 4-70 fold compared to the inactive state depending on the particular drug studied.

The data strongly suggests state dependent binding is necessary, but not sufficient for high affinity blockade of hERG. Further, the affinity of drugs for the S620T mutant represent their true affinity for the open conformation of the channel and the measured affinity for the WT channel is a weighted average of the affinity for the open and inactivated states. A corollary of our findings is that high throughput screens that do not assay inactive state binding may grossly underestimate the true affinity for WT-hERG channels.

Throughout this specification, unless the context requires otherwise, the word "comprise", or variations such as "comprises" or "comprising", will be understood to imply the inclusion of a stated element, integer or step, or group of elements, integers or steps, but not the exclusion of any other element, integer or step, or group of elements, integers or steps.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention as it existed in Australia before the priority date of each claim of this specification.

In order that the present invention may be more clearly understood, preferred embodiments will be described with reference to the following drawings and examples.

Brief Description of the Drawings

Figure 1: Schematic diagram of the S5, S6, pore helix, and S5P linker regions of two hERG subunits. The most important molecular determinants of drug-block are denoted with grey circles. Commonly employed mutants that disrupt inactivation are displayed with white stars. Note that G648 is probably not exposed to the pore cavity, and is thought to influence drug block by determining the conformation of the drug-binding pocket; V659 may influence drug-block by altering the gating characteristics of the channel. The S5P linker is dashed, as its exact position relative to the pore is unknown.

Figure 2: Conductance voltage curves for N588E-hERG (○), WT-hERG (■), and N588K-hERG (●). Data points are mean ± SEM and the curve fitted to the data is a Boltzmann function with a $V_{0.5}$ of inactivation of -114 ± 3 mV and slope factor of -19 mV (n=13) for N588E-hERG, $V_{0.5}$ of -78 ± 2 mV and slope factor of -26 mV (n= 11) for WT-hERG, and a $V_{0.5}$ of 45 ± 4 mV and slope factor of -14 mV (n=6) for N588K-hERG.

Figure 3: Typical examples of current traces recorded from the drug-block voltage protocol illustrated at top. A. WT-hERG, B. N588E-hERG, and C. N588K-hERG, in the presence of 30 nM cisapride. Dashed lines represent zero current in each case. Note the different scales on each recording; A. and B. are currents recorded during the revelatory voltage step, while C. displays the non-rectifying current of the 3s depolarising step in N588K-hERG. The percentage of drug-block for each recording was determined by dividing the current measured at the end of the 3s depolarising step to +20 mV after application of the drug ($I_{drug}$) by the current measured at the beginning of the recording ($I_{control}$). In the case of WT-hERG and N588E-hERG, the current at this point was determined by fitting an exponential curve to the first part of the deactivating current during the revelatory voltage step and extrapolating this back to the end of the depolarising step, as illustrated in A. and B.

Figure 4: Hill Plots of high-affinity hERG blockers. Drug binding to WT-hERG (■), N588E-hERG (○), and N588K-hERG (●) for A. astemizole, B. cisapride, C. dofetilide D. terfenadine. Each data point represents $I_{[drug]}$ / $I_{control}$ ± S.E.M for n= 4-9 cells. E. Summary data showing the mean $IC_{50}$ ± S.E.M for each drug. Significant differences between WT-hERG and N588K-hERG are denoted by *. All values are shown in Table 1.

Figure 5: Typical examples of current traces recorded from A. WT-hERG, B. N588E-hERG, and C. N588K-hERG in the presence and absence of 3 μM quinidine. The drug-block voltage protocol is shown at top. Dashed lines represent zero current in each case. Note the >50% of block of N588K-hERG by 3 μM quinidine which is similar to the percentage block in WT-hERG and N588E-hERG. Rates of deactivation in N588E-hERG are faster than WT-hERG due to the use of a -120 mV 'revelatory' voltage step, in order to produce adequate opening of channels to measure current. The rate of deactivation appears to slow in A. and B. with application of drug; this is probably due to some drug unblocking during this step, rather than a true effect on the kinetics of deactivation.

Figure 6: Hill plots of low affinity hERG blockers. Drug binding to WT-hERG (■), N588E-hERG (○), and N588K-hERG (●) for **A**. quinidine B. perhexiline C. erythromycin D. dl-sotalol. Each data point represents $I_{[drug]}$ / $I_{control}$ ± SEM for n= 4-6. E. Summary data showing the mean $IC_{50}$ ± S.E.M for each drug. Significant differences between WT-hERG and N588K-hERG are denoted by *. All values are shown in Table 1.

Figure 7: A. Conductance voltage curves for S631A-hERG(Δ), and S620T-hERG(□). Data points are mean ± SEM and the curve fitted to the data points is a Boltzmann function. The $V_{0.5}$ of steady state inactivation for the S631A-hERG mutant is 43 ± 2 mV with a slope factor of -16.5 mV (n=6). S620T-hERG does not inactivate in the voltage range shown. Dotted, plain, and dashed lines show data for N588E-hERG, WT-hERG, and N588K-hERG, reproduced from Figure 2. B. The affinity of dofetilide for S631A-hERG (Δ), and S620T-hERG (□). Dotted, plain, and dashed lines show data for N588E-hERG, WT-hERG, and N588K-hERG respectively, reproduced from Figure 4C. Note the similar drug affinity for the N588K and S631A cell constructs, and the marked right shift (reduced affinity) of dofetilide for the S620T construct.

Figure 8. Markov model of drug-binding to hERG. $C_x$ = closed states. O = open state. I = inactivated state. OD = drug bound to open state. ID = drug bound to inactivated state.

Figure 9: Dofetilide Hill curves with data points for N588K-hERG and the modelling curve superimposed. Thin plain line, dotted line, and dashed line show WT-, N588K-, and S620T-hERG respectively. The modelled line is thick black. Experimental $IC_{50}$ values for dofetilide block of N588K, and the modelled values respectively: 377 ± 57.8 vs 302 nM.

Figure 10: Hill curves for **A.** astemizole **B.** cisapride **C.** terfenadine and **D.** dl-sotalol with data points for N588K-hERG and the modelling curves superimposed. Thin plain line, dotted line, and dashed line show WT-, N588K-, and S620T-hERG

respectively. Modelled lines are thick black. Experimental $IC_{50}$ values for drug block of N588K and the modelled values respectively: astemizole - 14.8 ± 1.1 vs 13.6 nM; cisapride - 55.9 ± 4.2 vs 77.3 nM; terfenadine -170 ± 32.3 vs 177 nM; dl-sotalol 1392 ± 266.5 vs 1461 μM. $IC_{50}$ values for S620T are presented in Table 2.

Figure 11: Quinidine current clamp assay. Typical modelling experiments (a.), and raw data (b.), for quinidine current clamping experiments. In b. drug is applied at the beginning of the tracing, and results in depolarisation of the cell membrane. The magnitude of voltage change may be converted to a percentage drug block, and plotted on a standard Hill curve (c.) By this means the IC50 of drug block in the current clamp assay may be compared to the IC50 of drug block in the voltage clamp assay.

Figure 12: Synthetic hERG cDNA (1..3486). Contains possible mutations for enhancement of inactivation (G584S, H587K, N588E) and activation (L524W, L532P, V535A, A536V, K538Q. These mutations are underlined and the variable bases are indicated using the IUPAC codes. In addition there are multiple silent mutations introducing restriction sites.

In summary,
L524W (YKN) CTT, CTC, CTA, CTG, TTA, TTG, or TGG (SEQ ID NO 2)
L532P (YYN) CTT, CTC, CTA, CTG, TTA, TTG, CCT, CCC, CCA, or CCG (SEQ ID NO 3)
V535A (GYN) GTT, GTC, GTA, GTG, GCT, GCC, GCA, or GCG (SEQ ID NO 4)
A536V (GYN) GTT, GTC, GTA, GTG, GCT, GCC, GCA, or GCG (SEQ ID NO 5)
K538Q (MAR) AAA, AAG, CAA, or CAG (SEQ ID NO 6)
G584S (DSN) GGT, GGC, GGA, GGG, TCT, TCC, TCA, TCG, AGT, or AGC (SEQ ID NO 7)
H588K (MAN) CAT, CAC, AAA, or AAG (SEQ ID NO 8)
N588E (RAY) AAT, AAC, GAT, or GAC (SEQ ID NO 9)

IUPAC codes for nucleotides in sequence:

A Adenine
C Cytosine
G Guanine
T Thymine
U Uracil
R Purine (A or G)
Y Pyrimidine (C, T, or U)
M CorA
K T, U, or G
W T, U, or A
S C or G
B C, T, U, or G (not A)
D A, T, U, or G (not C)
H A, T, U, or C (not G)
V A, C, or G (not T, not U)
N Any base (A, C, G, T, or U)

Figure 13: Comparison of nucleotide sequence of synthetic construct) shown in Figure 12 (SEQ ID NO 1) to wild type hERG cDNA (SEQ ID NO 10).

Mode(s) for Carrying Out the Invention

The present invention is described herein using several definitions, as set forth below and throughout the specification.

As used herein, unless otherwise stated, the singular forms "a," "an," and "the" include plural reference. Thus, for example, a reference to "an oligonucleotide" includes a plurality of oligonucleotide molecules, and a reference to "a nucleic acid" is a reference to one or more nucleic acids.

As used herein, "about" means plus or minus 10%.

METHODS

**Molecular Biology**

Experiments on wild type (WT) hERG channels were performed using a Chinese hamster ovary (CHO) cell line stably expressing the hERG $K^+$ channel constructed as previously described (Walker BD et al., Br J Pharmacol 1999;128(2):444-450). CHO cells were cultured in D-ME/F12 with 10% FBS and maintained at 37°C in 5% $CO_2$. Cells were studied at least 24h after being plated on microscope coverslips. Mutant hERG constructs were generated using the Megaprimer PCR method. Mutant constructs (N588K, N588E, S631A or S620T) were sequenced to ensure that only the correct mutation had been made and then subcloned into the pIRES2-eGFP vector. Blank CHO cells were plated onto sterilised glass coverslips. After 24h, the cells were transfected with mutant hERG constructs using PolyFect Transfection Reagent (Qiagen) according to the manufacturer's instructions. After transfection, the cells were incubated at 37°C for a minimum of 1 day prior to electrophysiological study. Successfully transfected cells, identified by the expression of eGFP, were studied using the whole-cell configuration of the patch clamp technique.

## Electrophysiology

Borosilicate glass tubing patch pipettes, with resistances of 1-4 MΩ when filled with internal solution, were made using a vertical 2-stage puller (PP-830; Narishige, Japan). The internal solution contained (in mM) 120 potassium gluconate, 20 KCl, 1.5 MgATP, 5 EGTA and 10 HEPES (pH 7.3 with KOH). Membrane potentials where adjusted by -15 mV to correct for the junction potential (Barry, 1994) between low $Cl^-$ pipette solution and external bath solution (see below). Currents were amplified (Axopatch 200B amplifier; Molecular Devices, Sunnyvale, Ca) and digitised (DigiData 1200; Molecular Devices) before storage on a personal computer. Capacitance current transients were electronically subtracted, and series resistance compensation was ~80% for all experiments. Current signals were digitised at 5kHz and low-pass filtered at 2KHz. Some current traces were leak-subtracted off-line. Acquisition was performed using pClamp software.

## Solutions and Drugs

Cells were superfused with a Tyrode solution containing (in mM): 130 NaCl, 5 KCl, 1 MgCl, 1 $CaCl_2$, 12.5 Glucose, 10 Hepes. Cisapride, Astemizole, Terfenadine, Erythromycin, Dofetilide, and Quinidine, were prepared as stock solutions in dimethyl sulphoxide (DMSO) and subsequently diluted as required with superfusate (maximum final DMSO concentration = 0.1% $vv^{-1}$). Dl-Sotalol was prepared as a stock solution in Tyrode solution, and Perhexiline as a stock solution in methanol. We have previously found that DMSO at 0.1% $vv^{-1}$ has no effect on the parameters under study.

## Voltage Protocols

*Steady State Inactivation*

From a holding potential of -80 mV the membrane voltage was stepped to +40 mV for 3 seconds to fully activate the channels. For cells expressing WT-hERG and N588E-hERG, the second pulse consisted of a voltage step from +40 mV to -160 mV in 10 mV decrements. For cells expressing N588K, a range of +120 mV to -20 mV was employed. For voltage steps to < -30 mV, an exponential curve was fitted to the first phase of deactivation and extrapolated back to the beginning of the second pulse at which point its magnitude represents the current that the channel would have passed if recovery from inactivation were instantaneous. Channel conductance was plotted against voltage to yield the voltage-dependent steady state inactivation curve (properly, the steady state recovery from inactivation) and the data fitted with a Boltzmann function:

$$\frac{G}{G_{max}} = \frac{1}{(1+e^{\frac{V_{0.5}-V_t}{k}})}$$

$V_{0.5}$ - half inactivation voltage

k - slope factor

*Drug Block*

As this was a comparative study, care was taken to ensure similar conditions for drug testing in WT and mutant channels. However, due to the unique gating characteristics of N588E-hERG and N588K-hERG, slight alterations in the voltage protocol and method of measurement were used. Currents were measured with a two-step voltage protocol; a first step to +20 mV for 3 seconds to fully activate the channels and a second step to a negative membrane potential, usually -110 mV (but see below for variations). During this second voltage step hERG channels quickly recover from inactivation and proceed to deactivate. This second step was termed 'revelatory', because it allowed us to estimate the total conductance of activated channels (open and inactive) after the 3s period of depolarisation.

The revelatory step was typically recorded at -110 mV, although in some N588E-hERG cells a voltage step to -120 mV was employed to allow adequate recovery from inactivation for current measurement. Conversely, a less negative voltage was used for a few N588K-hERG cells in order to minimise series resistance errors due to the large activating current in this non-rectifying construct. Drug block was calculated as $I_{[drug]}/I_{control}$, with all currents measured at the end of the activating step. For N588E-hERG and WT-hERG a single exponential fit was applied to the first part of the current trace during the revelatory step and extrapolated back to the end of the activating step. In this way current was measured at the same time point for all cells.

Voltage-protocols were repeated at 0.1 Hz. Control currents were recorded 3-5 minutes after patch rupture, i.e. the time take for current levels to stabilise at a steady state level. The first drug was applied, with solution exchange typically taking less than 10s. Recording continued until a new steady state block was reached (typically 2-4 minutes). Between 2 and 4 doses of drug were applied to each cell with most experiments completed within 20 minutes.

Data Analysis

Initial data analysis was performed using the Clampfit module of the pClamp 9.0 software. Subsequent data analysis and preparation of data for figures were performed with Mathematica 6 (Wolfram Technologies). All data are expressed as mean $\pm$ SEM (n) and statistical significance ($P<0.05$) was determined using paired t-tests.

Vectors

hERG cDNA was cloned into a mammalian expression construct containing a CMV viral promoter to direct expression and a G418 antibiotic selection cassette to permit selection of stable cell lines. Stable cell lines were generated as previously described (Walker BD et al., Br J Pharmacol 1999;128(2):444-450). It will be appreciated that any suitable vector known to the art would be suitable.

**Host Cells**

The preferred host cell is Chinese hamster ovary cells, human embryonic kidney cells or transformed African Green Monkey kidney fibroblast cells (COS7). Techniques for obtaining stable host cells expressing a mutant protein are well known to the art and can be found from online protocols under the Biology-Transfection-Stable-Transfection online document used by persons in this art.

**Screening Assays**

The mutant hERG constructs and/or stable cell lines can be assayed using conventional patch clamp electrophysiology assays (Perrin MJ et al, Mol Pharmacol. 2008 Nov; 74(5):1443-52), Current clamp assays from moleculardevices, Rb+ flux assays (Rezazadeh S et al., J Biomol Screen. 2004 Oct;9(7):588-97) or voltage following assays utilizing voltage sensitive dyes (Dorn A et al, J Biomol Screen. 2005 Jun; 10(4):339-47).

RESULTS

**$V_{0.5}$ of Steady State Inactivation in WT-hERG, N588E-hERG and N588K hERG expressed in CHO cells**

To investigate the link between drug binding and state dependence we chose to use mutants of residue N588 located in the $\alpha$-helix of the S5P linker of hERG (Figure 1). This residue has two important features, firstly, it is though to be located distant to the drug binding pocket and secondly it is possible to titrate the voltage-dependence of inactivation of the channels by introducing different charges at this residue.

Studies of N588 mutant channels have been carried out using the *Xenopus laevis* oocyte expression system; however, mammalian cells are the preferred heterologous expression system to use for drug binding studies. Therefore, we first wanted to confirm that the properties of N588 mutant channels were similar in mammalian cells and Xenopus laevis oocytes. When expressed in CHO cells, the $V_{0.5}$ of inactivation for N588E-hERG was -114 mV $\pm$ 3 mV with a slope factor of -19 mV $\pm$ 1 mV, (n=13); the $V_{0.5}$ of inactivation for WT-hERG was -78 mV $\pm$ 2 mV with a slope factor of -27 mV $\pm$ 2 mV, (n=11); and the $V_{0.5}$ of inactivation for N588K-hERG was 45 mV $\pm$ 4 mV with a slope factor of -14 mV $\pm$ 1 mV, (n=6) (Figure 2). All values are similar to those reported for Xenopus oocytes. The reversal potential for N588K-hERG; -83.4 $\pm$ 2.0 mV (n=5), was slightly shifted compared to that for WT-hERG; -88.5 $\pm$ 2.2 mV, n=10. The N588K-hERG channels are nevertheless still highly selective for $K^+$ over $Na^+$. The reversal potential in the N588E-hERG construct could not be determined due to the small magnitude of its activating currents. However, when expressed in oocytes with much larger currents it was found to be similar to WT-hERG. These properties together make N588 mutant constructs ideal for the investigation of inactivation mediated drug-binding to hERG.

*High Affinity Drug Binding is modulated by N588 charge mutants*

We initially investigated the affinity of 4 drugs, previously established to block hERG in the low nanomolar range - astemizole, cisapride, dofetilide, and terfenadine, for N588E-hERG, WT-hERG and N588K-hERG expressed in CHO cells. Figure 3 shows typical examples of WT-, N588E- and N588K-hERG traces under control conditions and after 5 minutes equilibration with 30 nM cisapride. Percentage of drug-block was measured at the end of the 3s-activating step to +20 mV in all cells. This was performed directly in N588K (Figure 3c), or else by fitting a single exponential curve to the first part of the current trace during the revelatory step in N588E-hERG (Figure 3b) and WT-hERG (Figure 3a) and extrapolating this back to the end of the activating step. The data in Figure 3 indicate that 30 nM cisapride caused less block of N588K-hERG channels compared to N588E- or WT-hERG. This is more clearly seen from the summary Hill-plots shown in Figure 4; cisapride affinity for WT=hERG: 20.5 ± 2.2 nM (n=4) was similar to that for N588E-hERG: 13.1± 4.9 nM (n=4) but much reduced for N588K-hERG: 55.9 ± 4.2 nM (n=4). All four high-affinity blockers showed a similar pattern; reduced affinity for N588K-hERG compared to WT- and N588E-hERG (see Figure 4). The affinities for all drugs for WT and N588 mutant constructs are summarised in Table 1.

Table 1 - Affinities for all drugs for WT and N588 mutant constructs

| Drug | N588E | WT-hERG | N588K |
|---|---|---|---|
| Astemizole | 4.8 ± 1.2 nM (5); h=1.2 | 5.1 ± 1.4 nM (6); h= 1.0 | 14.8 ± 1.1 nM (5)+*; h= 0.8 |
| Cisapride | 13.9 ± 4.9 nM (4); h = 0.8 | 20.5 ± 2.2 nM (6); h = 1.0 | 55.9 ± 4.2 nM (6)+*; h=0.9 |
| Dofetilide | 39.4 ± 7.3 nM (5); h = 1.1 | 51.0 ± 1.3 nM (9); h = 1.3 | 377.3 ± 57.8 nM (5) +*; h = 1.3 |
| Terfenadine | 48.2 ± 10.6 nM (5); h=1.0 | 61.4 ± 16.2 nM (9); h = 1.2 | 170.2 ± 32.3 nM (7) +*; h=0.9 |
| Quinidine | 3.71 ± 0.4 μM (4); h = 1.1 | 3.2 ± 0.3 μM (5); h = 0.8 | 3.9 ± 0.3 μM (4); h = 0.9 |
| Perhexiline | 5.0 ± 1.8 μM (5); h = 0.8 | 5.9 ± 0.9 μM (7); h = 1.1 | 4.7 ± 1.7 μM (5); h = 1.2 |
| Erythromycin | 98.4 ± 39.7 μM (5); h = 0.8 | 129.5 ± 59.9 μM (4); h = 0.7 | 151.1 ± 49.4 μM (4); h=0.8 |
| dl-Sotalol | 585.6 ± 178.6 μM (4); h = 0.8 | 515.5 ± 35.5 μM (4); h=0.9 | 1392.0 ± 266.5 μM (4)+*; h = 0.9 |

*Low Affinity Drug Binding to N588 charge mutants*

We next investigated the affinity of 4 drugs previously established to block hERG in the high nanomolar or micromolar range - quinidine, perhexiline, erythromycin, and dl-sotalol, for the N588E-hERG, WT-hERG and N588K-hERG constructs expressed in CHO cells. Typical examples of current traces recorded from WT-, N588K- and N588E-hERG in the presence and absence of 3μM quinidine are illustrated in Figure 5. Quinidine, 3 μM, caused a similar degree of block of WT and the two N588 charge mutants. This is also seen from the summary Hill plots (Figure 6A). Perhexiline and erythromycin showed the same pattern as that observed for quinidine, i.e. similar affinities for all hERG constructs (Figure 6A, C). In contrast, dl-sotalol showed a significantly higher affinity for N588E-hERG and WT-hERG compared to N588K-hERG (Figure 6D, Table 1).

**Reduced affinity for N588K-hERG and state-dependent binding**

The data from Figures 3 and 4 clearly demonstrate that the four high-affinity drugs used in this study showed reduced affinity for the inactivation-deficient N588K-hERG channels. To determine whether this reduced affinity for N588K-hERG reflected a state-dependence of drug-binding, we investigated whether there was a similar reduced affinity for a range of inactivation-deficient mutants. Specifically, we investigated binding of dofetilide to S631A-hERG and S620T-hERG. S631A-hERG has a markedly right-shifted $V_{0.5}$ of steady state inactivation compared to WT-hERG that is very similar to that observed for N588K (Figure 7A) whereas S620T-hERG does not inactivate at measurable voltages. Accordingly, at +20 mV, the proportion of channels in the Open:Inactivated states is -95:5 for N588K and S631A, compared to 100:0 for S620T, but 5:95 for WT-hERG (Figure 7A).

The affinity of dofetilide for S631A-hERG was not statistically different to that for N588K-hERG (404 ± 95 nM (n=4), and 377 ± 69 nM (n=4) respectively), an 8-fold reduction compared to WT-hERG. That these two mutants, with very similar effects on inactivation but apparently not located near each other, have very similar effects on drug binding suggests that the reduced affinity for drug binding is mediated by the reduced inactivation of the channel. However, the affinity of dofetilide for S620T, was reduced a further 10 fold (3494 nM ± 374 nM, n=4) (Figure 7B) compared to its affinity for S631A or N588K. Given that there is relatively little difference in the extent to which S631A and N588K channels occupy the open state at +20 mV (the voltage at which we assayed drug binding) compared to S620T channels (i.e. ~85% compared to 100%), a marked reduction in drug-affinity for the S620T-hERG suggests a direct effect on drug binding by this mutant. An alternative hypothesis is that, despite the relative infrequency with which S631A and N588K channels occupy the inactive state at +20 mV, the kinetics of drug binding and unbinding are such that whenever the channel enters the inactive state it binds drug that, with a very slow off rate, remains bound for a long period. According to this hypothesis, binding of drug to the S620T mutant would only encounter the open state and so reflect the affinity for the open state, whereas binding to WT or N588K channels would reflect a weighted average of the affinity for the inactive and open states dependent on the relative rates of transitions between the two states and drug binding and unbinding rates. To test this hypothesis we set up a computer model of drug binding to hERG channels as depicted in Figure 8.

**Modelling kinetics of drug binding to open and inactive states**

The Markov chain model for hERG kinetics is based on that developed by Lu et al. (J Physiol. 2001 Dec 15; 537(Pt 3):843-51) with the addition of two more states: drug-bound open state and drug-bound inactivated state (Figure 8). The rate constants, scaled to 22°C, for the model are shown in the data supplement. To model the S620T mutant the rate constants for transition steps to the inactivated state were set to 0. To model the N588K mutant, the rate constants for the transition between the open and inactive states were adjusted to reproduce the experimentally observed changes in these rate constants and the steady-state inactivation. For simplicity, we assumed that the kinetics of activation were the same for WT, S620T and N588K channels.

To calculate the rate constants for open and inactive state drug blocked we assumed that drug affinity for S620T represented the affinity for the open state. The rate constants for open state drug block, kf6 and kr6, accordingly were constrained to fit the data for drug binding to the S620T channels. The values for kf6 and kr6 were then held constant and the values for drug binding to the inactivated state, kf7 and kr7, constrained to fit the time course of drug block for WT channels. In the case of dofetilide binding, the calculated affinity for the open state (kf6/kr6) was 3.5 $\mu$M, i.e. the value for S620T, and the calculated affinity for the inactive state (kf7/kr7) was 47.8 nM. The measured affinity for dofetilide binding to WT channels, 50.1 nM, is much closer to the calculated value for the affinity to the inactive state reflecting both the greater proportion of time WT channels spend in the inactive state and the slower dissociation of drug from the inactive state. If the original assumption was correct, i.e. drug affinity for S620T is the true affinity of the drug for the open state, then substitution of the values for kf6, kr6, kf7 and kr7 into our model for N588K should reproduce the experimentally determined $IC_{50}$ for dofetilide binding to N588K. As can be seen from the data in Figure 9, the model predicted values are very close to the experimental data. The same procedure was repeated for each of the state-dependent drugs formerly assessed (Figure 10A-D) with similarly good approximations to the experimental data.

Incorporation of the model for hERG drug binding into a CHO-cell model of membrane potential was also able to reproduce the effects of drugs on membrane potential in a hERG transfected CHO-cell. In a blank CHO cell the resting membrane potential is --30 mV compared to --60 mV for the CHO cell line stably transfected with the hERG K+ channel. 50% block of the hERG channels causes a 3.5 mV increase in membrane potential. This agrees very well with the experimental data (Figure 11). Furthermore, modelling studies indicate that the change in membrane potential can be enhanced from 3.5 mV to 4.5 mV per 50% reduction in hERG channel activity by shifting the voltage-dependence of activation by -15 mV.

DISCUSSION

**State-dependence of drug binding**

Most drugs that block hERG require channel opening. Some evidence suggests that, once open, inactivation increases drug affinity for the channel: first, mutant hERG channels with disrupted inactivation - S631A, S620T, and G628C/S631C reduce drug-block of hERG by multiple agents. Furthermore, mutations introduced into Bovine-EAG (bEAG) that enable channel inactivation also confer sensitivity to dofetilide channel block. However, the inactivation disrupting mutations employed could affect drug-block through gating independent means. S631 and S620 lie proximate to the drug-binding space, and may produce conformational changes at the base of the pore helix - an area known to be an important molecular determinant of drug-binding. G628C/S631C markedly reduces the potassium selectivity of hERG, suggesting significant conformational changes in the vicinity of the selectivity filter have taken place in this mutant. Further, the mutant hERG channels G648A and S623A both promote inactivation, but reduce methanesulfonamide block of hERG. Adding to the difficulty of interpreting whether drug binding is state-dependent, voltage protocols designed to favour occupancy of the inactivated state during drug-binding (i.e. large depolarisations) also relieve drug block.

The data reported in this application provides the strongest evidence to date that drug binding to hERG K+ channels can be state dependent. Firstly, we have shown that a mutation introduced into a region thought to be remote from the drug binding pocket yet still affects inactivation (N588K) affects drug binding. In the absence of a crystal structure of hERG in open and inactive states we cannot be certain that the position of N588K is remote from the drug binding pocket, however a combination of structural studies, toxin binding studies and molecular dynamics modelling studies suggest that N588 on the hydrophilic surface of the S5P $\alpha$-helix will be facing the extracellular space and therefore well away from the drug binding pocket. Secondly, we have shown that two distinct mutants that have very similar effects on inactivation (N588K and S631A) have the same effect on drug binding. The simplest explanation for this observation is that it is the effect these mutations have on inactivation that accounts for the altered drug binding. Thirdly, our kinetic modelling of drug binding to WT, S620T and N588K mutant channels shows that all the differences in drug binding between these mutants can be explained simply on the basis of differences in occupancy of open and inactive states and the kinetics of drug binding and unbinding. An important corollary of our kinetic modelling studies is that for the first time we have been able to estimate the affinities for both the open and inactive states for a range of drugs. Furthermore, our data suggest that the affinities for the open and inactive states of any drug can now be calculated if one knows the affinity for WT and S620T mutant channels.

In contrast with our data, there is one report in the literature that suggests that the S620T mutation could have a direct effect on drug binding. It was shown that S620T and S620C, whilst having the same effects on inactivation gating (abolition), had different affinities for E-4031 - a methanesulfonamide similar to dofetilide. This was interpreted as indicating that the Serine side chain at 620 contributed directly to drug binding. This data, however, is not necessarily incompatible with our data. It is possible that the abolition of inactivation alters drug affinity in both the S620T and S620C but that the cysteine side chain in S620C, but not the threonine side chain in S620T, can bind to the drug in the open state and thereby increase the affinity relative to that observed for S620T. Further, drug block in their studies was assessed after a 4s depolarising step to + 50 mV. At this voltage the O:I probability for S631A should be ~ 1:1, significantly different to S620C which does not inactivate. However, the affinity of E-4031 for S631A- and S620C-hERG was similar suggesting that one or the other alters drug block with some independence from gating mechanisms. Our data for dofetilide block of S631A and N588K, i.e. S631A affects drug block by altered gating, implies that it is S620C which exerts a direct effect on drug binding.

**Relevance for high throughput screens for drug binding to hERG**

Given the mandated need to screen all drugs for hERG binding, there has been considerable effort put into developing high throughput screens for assaying drug binding to hERG. Such assays have included fluorescence based voltage assays and Rb+ efflux assays. In general, however, the results of these screens have been poor and we suggest that this is because they predominantly assay binding to the open state and therefore fail to detect many if not all high affinity blockers. Our data show that the difference in affinity between the open and inactive states can be 70-fold. Therefore, it is imperative that to be successful any high throughput screening system adequately assay binding to the inactive state.

Our modelling studies also indicate that mutations that result in a leftward shift in the voltage-dependence of activation will increase the voltages shifts caused by drug block of hERG channels and thereby facilitate high throughput drug screening assays based on measuring changes in voltage.

OBSERVATIONS

We investigated the relationship between inactivation gating and drug-block of hERG, finding that high affinity block (nanomolar range) is promoted by inactivation. The use of charged mutants at N588 provides a methodology for investigating the conformational changes of the channel pore between open and inactivated states. Further, we have calculated for the first time the relative affinities of drug binding to the open and inactive states of the hERG channel, which in the case of dofetilide shows a 70-fold greater affinity for the inactive state. The pharmaceutical importance of these data is highlighted by the observation that two drugs that have been withdrawn from the market; astemizole and terfenadine and one that has had its use severely restricted; cisapride) exhibit a marked preference for binding to the inactive state.

It will be appreciated by persons skilled in the art that numerous variations and/or modifications may be made to the invention as shown in the specific embodiments. The present embodiments are, therefore, to be considered in all respects as illustrative and not restrictive.

<u>Numbered Disclosure</u>

The following numbered disclosure also form part of the description.

1. An isolated nucleic acid molecule capable of expressing an human ether-à-go-go related gene (hERG) mutant that enhances hERG inactivation, wherein the mutation is not located in the vicinity of the drug-binding pocket within the cavity of the ion conduction pore of the hERG protein.
2. The nucleic acid molecule according to embodiment 1 wherein the mutation is located on the alpha-helix of the S5P linker.

3. The nucleic acid molecule according to embodiment 2 wherein the mutation is N588E, H587K, G584S, or Q592D.

4. The nucleic acid molecule according to embodiment 3 wherein the mutation is N588E.

5. The nucleic acid molecule according to embodiment 1 containing one or more mutations as substantially as set out in Figure 12 (SEQ ID NO: 1).

6. The nucleic acid molecule according to any one of embodiments1 to 4 further containing one or more mutations selected from L524W, L532P, V535A, A536V or K538Q that promotes activation of the hERG ion channel.

7. A vector for expressing an isolated nucleic acid molecule according to any one of embodiments 1 to 6.

8. The vector according to embodiment 7 containing a promoter suitable for expression in a mammalian cell and an antibiotic resistance gene to aid selection of cells expressing the hERG channel.

9. A stable cell line expressing an isolated nucleic acid molecule according to any one of embodiments 1 to 6.

10. A stable cell line containing a vector according to embodiments 7 or 8 and expressing a hERG mutant protein.

11. The stable cell line according to embodiments 8 or 9 selected from Chinese hamster ovary cell, human embryonic kidney cell or transformed African Green Monkey kidney fibroblast cell.

12. An assay for screening potential drug binding to hERG protein comprising:

a stable cell line according to any one of embodiments 9 to 11; and
means to determine activity of the hERG protein in the cell.

## Claims

1. An assay for screening potential drug binding to hERG protein, the assay comprising:

   providing a stable mammalian cell line containing a nucleic acid molecule expressing an human ether-à-go-go related gene (hERG) N588E mutant that enhances hERG inactivation of the cell;
   incubating a test drug with the stable cell line; and
   determining activity of the hERG mutant protein by the effects on ion flux or voltage in the stable mammalian cell line.

2. The assay according to claim 1 wherein the stable mammalian cell line expresses a hERG mutant further comprising the mutations G584S, Q592D, or a combination thereof.

3. The assay according to claim 1 or 2 wherein the stable mammalian cell line contains one or more hERG mutations as set out in SEQ ID NOS: 2 to 9.

4. The assay according to any one of claims 1 to 3 wherein the stable mammalian cell line further expresses one or more hERG mutations selected from L524W, L532P, V535A, A536V or K538Q that promotes activation of the hERG ion channel.

5. The assay according to any one of claims 1 to 4 wherein the stable mammalian cell line contains a vector for expressing the nucleic acid molecule encoding the hERG mutant.

6. The assay according to claim 5 wherein the vector further contains a promoter suitable for expression in a mammalian cell and an antibiotic resistance gene to aid selection of cells expressing the hERG channel.

7. The assay according to any one of claims 1 to 6 wherein the cell line is selected from Chinese hamster ovary cell, human embryonic kidney cell or transformed African Green Monkey kidney fibroblast cell.

8. The assay according to any one of claims 1 to 7 wherein the hERG activity of the cell is determined by ion flux or voltage changes using patch clamp assays, Rb+ efflux assays as a surrogate for K+ flux or fluorescence to assay voltage changes of the cell.

9. A stable mammalian cell line containing an expression vector containing an isolated nucleic acid molecule expressing a hERG mutant containing the N588E mutation as set out in SEQ ID NO 9 that enhances hERG inactivation in a cell.

10. The stable mammalian cell line according to claim 9 selected from Chinese hamster ovary cell, human embryonic kidney cell or transformed African Green Monkey kidney fibroblast cell.

11. The stable mammalian cell line according to claim 9 wherein the vector further contains a promoter suitable for expression in the mammalian cell and an antibiotic resistance gene to aid selection of cells expressing the hERG channel.

**12.** The stable mammalian cell line according to claim 9 wherein the isolated nucleic acid further expresses one or more hERG mutations selected from L524W, L532P, V535A, A536V or K538Q that enhance activation of the hERG ion channel.

**Patentansprüche**

**1.** Testverfahren zum Durchsuchen einer möglichen Arzneimittelbindung an das hERG-Protein, wobei das Testverfahren umfasst:

Bereitstellen einer stabilen Säugetierzelllinie, die ein Nukleinsäuremolekül enthält, das eine N588E-Mutante des auf den Menschen bezogenen Ether-ä-go-go-Gens (human ether-ä-go-go related gene (hERG)) enthält, das die hERG-Inaktivierung der Zelle vergrößert;
Inkubieren eines Testarzneimittels mit der stabilen Säugetierzelllinie; und
Bestimmen der Aktivität des Proteins der hERG-Mutante durch die Wirkungen auf den Ionenfluss oder auf die Spannung in der stabilen Säugetierzelllinie.

**2.** Testverfahren nach Anspruch 1, wobei die stabile Säugetierzelllinie eine hERG-Mutante exprimiert, die ferner die Mutationen G584S, Q592D oder eine Kombination derselben umfasst.

**3.** Testverfahren nach Anspruch 1 oder 2, wobei die stabile Säugetierzelllinie eine oder mehrere hERG-Mutationen, wie sie in SEQ ID NOS: 2 bis 9 festgelegt sind, enthält.

**4.** Testverfahren nach einem der Ansprüche 1 bis 3, wobei die stabile Säugetierzelllinie ferner eine oder mehrere hERG-Mutationen exprimiert, die aus L524W, L532P, V535A, A536V oder K538Q ausgewählt sind und die die Aktivierung des hERG-Ionenkanals fördern.

**5.** Testverfahren nach einem der Ansprüche 1 bis 4, wobei die stabile Säugetierzelllinie einen Vektor enthält, um das Nukleinsäuremolekül, das die hERG-Mutante codiert, zu exprimieren.

**6.** Testverfahren nach Anspruch 5, wobei der Vektor ferner einen Promotor, der für eine Expression in einer Säugetierzelle geeignet ist, und ein gegenüber Antibiotika resistentes Gen enthält, um bei der Auswahl von Zellen, die den hERG-Kanal exprimieren, zu helfen.

**7.** Testverfahren nach einem der Ansprüche 1 bis 6, wobei die Zelllinie aus der chinesischen Hamstereizelle, aus der menschlichen embryonalen Nierenzelle oder aus der transformierten Nierenfibroblastzelle des Afrikanischen Grünen Affen (African Green Monkey) ausgewählt ist.

**8.** Testverfahren nach einem der Ansprüche 1 bis 7, wobei die hERG-Aktivität der Zelle durch Änderungen des Ionenflusses oder der Spannung unter Verwendung von Patch-Clamp-Testverfahren, (Rb+)-Ausstromtestverfahren als ein Ersatz für einen (K+)-Fluss oder unter Verwendung von Fluoreszenz, um Spannungsänderungen der Zelle zu testen, bestimmt wird.

**9.** Stabile Säugetierzelllinie, die einen Expressionsvektor enthält, der ein isoliertes Nukleinsäuremolekül enthält, das eine hERG-Mutante exprimiert, die die N588E-Mutation, wie sie in SEQ ID NO 9 festgesetzt ist und die die hERG-Inaktivierung in einer Zelle vergrößert, enthält.

**10.** Stabile Säugetierzelllinie nach Anspruch 9, die aus der chinesischen Hamstereizelle, der menschlichen embryonalen Nierenzelle oder aus der transformierten Nierenfibroblastzelle des Afrikanischen Grünen Affen (African Green Monkey) ausgewählt ist.

**11.** Stabile Säugetierzelllinie nach Anspruch 9, wobei der Vektor ferner einen Promotor, der für eine Expression in der Säugetierzelle geeignet ist, und ein gegenüber Antibiotika resistentes Gen enthält, um bei der Auswahl von Zellen, die den hERG-Kanal exprimieren, zu helfen.

**12.** Stabile Säugetierzelllinie nach Anspruch 9, wobei die isolierte Nukleinsäure ferner eine oder mehrere hERG-Mutationen exprimiert, die aus L524W, L532P, V535A, A536V oder K538Q ausgewählt sind und die die Aktivierung des hERG-Ionenkanals fördern.

**Revendications**

1. Dosage pour le criblage d'une liaison d'un médicament potentiel avec une protéine de hERG, le dosage comprenant les étapes consistant à :

   mettre en oeuvre une lignée cellulaire stable de mammifère contenant une molécule d'acide nucléique exprimant un mutant de gène apparenté d'éther-à-go-go humain (hERG) N588E, qui renforce l'inactivation de hERG de la cellule ;
   incuber un médicament d'essai avec la lignée cellulaire stable ; et
   déterminer l'activité de la protéine mutante de hERG par les effets sur le flux ou le potentiel ionique dans la lignée cellulaire stable de mammifère.

2. Dosage selon la revendication 1, dans lequel la lignée cellulaire stable de mammifère exprime un mutant de hERG comprenant en outre les mutations G584S, Q592D ou une de leurs combinaisons.

3. Dosage selon la revendication 1 ou la revendication 2, dans lequel la lignée cellulaire stable de mammifère contient une ou plusieurs mutations de hERG telles qu'établies dans les SEQ ID n° 2 à 9.

4. Dosage selon l'une quelconque des revendications 1 à 3, dans lequel la lignée cellulaire stable de mammifère exprime en outre une ou plusieurs mutations de hERG choisies entre L524W, L532P, V535A, A536V ou K538Q qui favorisent l'activation du canal ionique de hERG.

5. Dosage selon l'une quelconque des revendications 1 à 4, dans lequel la lignée cellulaire stable de mammifère contient un vecteur pour exprimer la molécule d'acide nucléique codant pour le mutant de hERG.

6. Dosage selon la revendication 5, dans lequel le vecteur contient en outre un promoteur qui convient à une expression dans une cellule de mammifère et un gène de résistance aux antibiotiques pour favoriser la sélection de cellules exprimant le canal de hERG.

7. Dosage selon l'une quelconque des revendications 1 à 6, dans lequel la lignée cellulaire est choisie entre une cellule ovarienne de hamster chinois, une cellule de rein embryonnaire humaine ou une cellule de fibroblaste de rein de singe vert d'Afrique.

8. Dosage selon l'une quelconque des revendications 1 à 7, dans lequel l'activité de hERG de la cellule est déterminée par des changements de flux ou de potentiel ionique en utilisant des dosages de type « patch clamp », des dosages d'efflux de Rb+ comme substitut pour un flux ou une fluorescence de K+ aux changements de potentiel de dosage de la cellule.

9. Lignée cellulaire stable de mammifère contenant un vecteur d'expression contenant une molécule d'acide nucléique isolée exprimant un mutant de hERG contenant la mutation N588E telle qu'établie dans la SEQ ID n° 9 qui renforce l'inactivation de hERG dans une cellule.

10. Lignée cellulaire stable de mammifère selon la revendication 9 choisie entre une cellule ovarienne de hamster chinois, une cellule de rein embryonnaire humaine ou une cellule de fibroblaste de rein de singe vert d'Afrique.

11. Lignée cellulaire stable de mammifère selon la revendication 9, dans lequel le vecteur contient en outre un promoteur qui convient à une expression dans la cellule de mammifère et un gène de résistance aux antibiotiques pour favoriser la sélection de cellules exprimant le canal de hERG.

12. Lignée cellulaire stable de mammifère selon la revendication 9, dans lequel l'acide nucléique isolé exprime en outre une ou plusieurs mutations de hERG choisies entre L524W, L532P, V535A, A536V ou K538Q qui favorisent l'activation du canal ionique de hERG.

# Figure 1

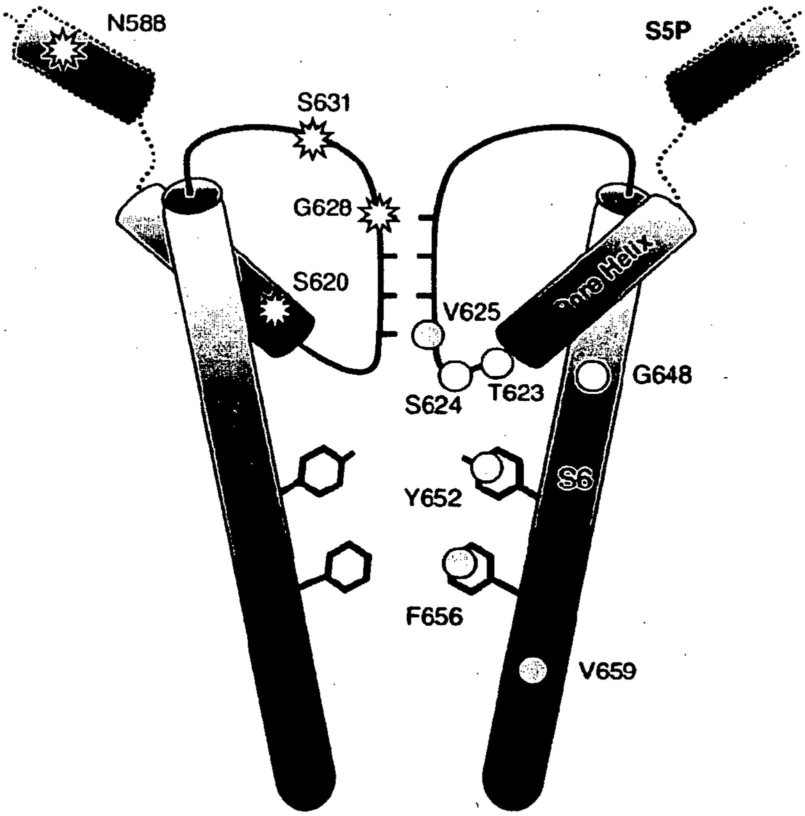
N588
S5P
S631
G628
S620
V625
Pore Helix
G648
S624
T623
Y652
S6
F656
V659

**Figure 2**

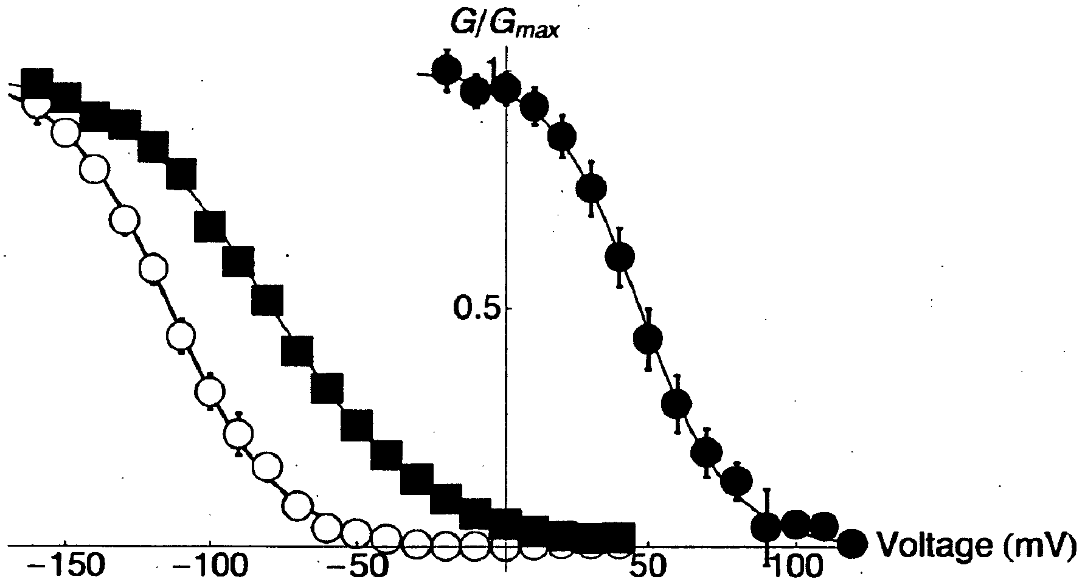

# Figure 3

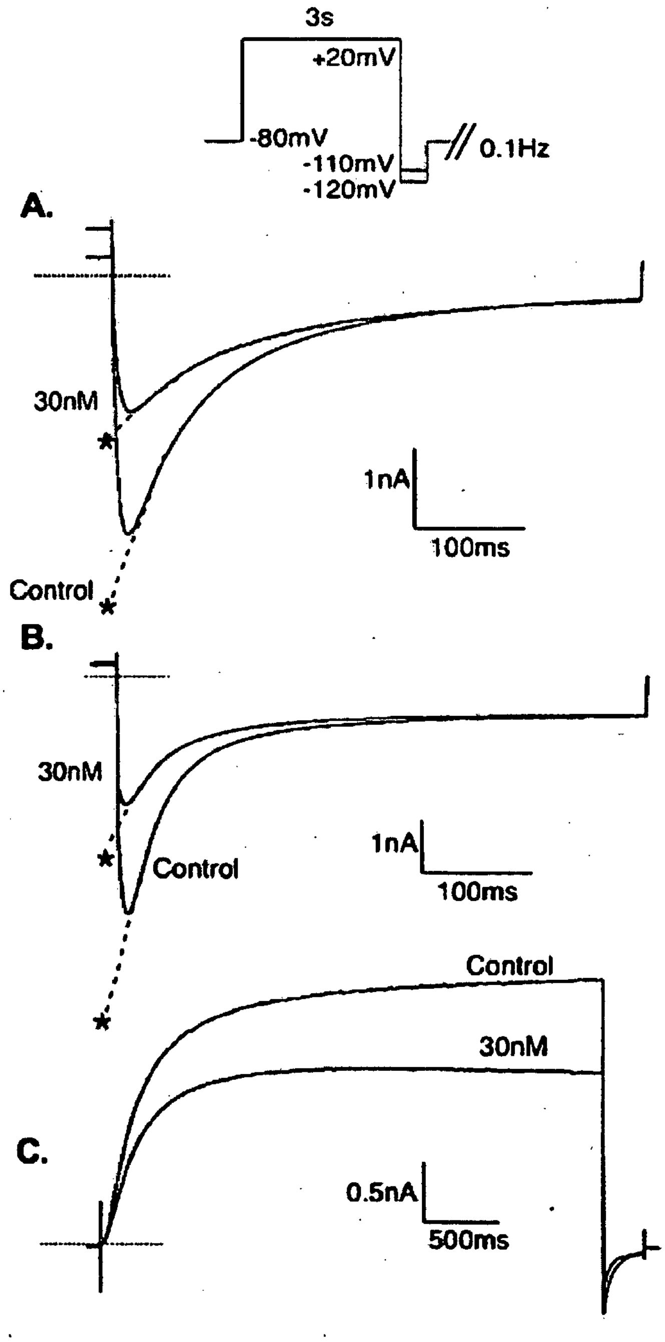
3s
+20mV
-80mV
-110mV
-120mV
0.1Hz
A.
30nM
Control
1nA
100ms
B.
30nM
Control
1nA
100ms
Control
30nM
C.
0.5nA
500ms

# Figure 4

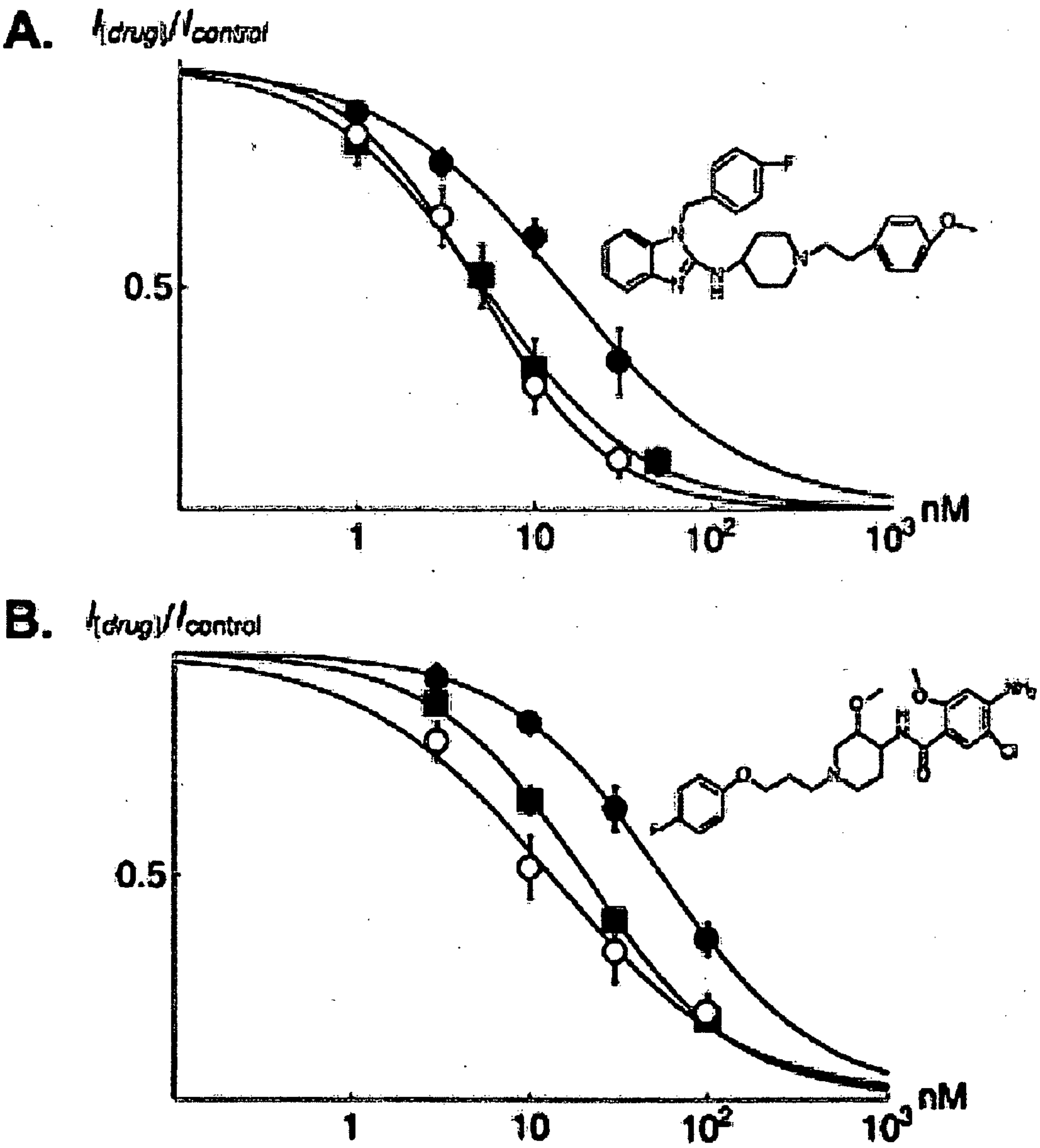
A.
I(drug)/I control
0.5
1
10
10²
10³
nM
B.
I(drug)/I control
0.5
1
10
10²
10³
nM

# Figure 4 (cont.)

C.

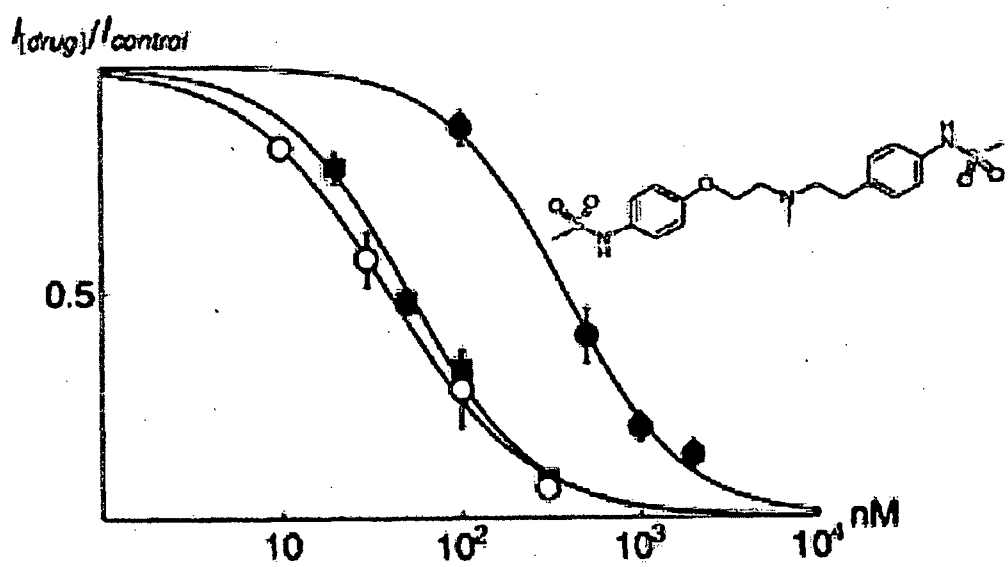


D.

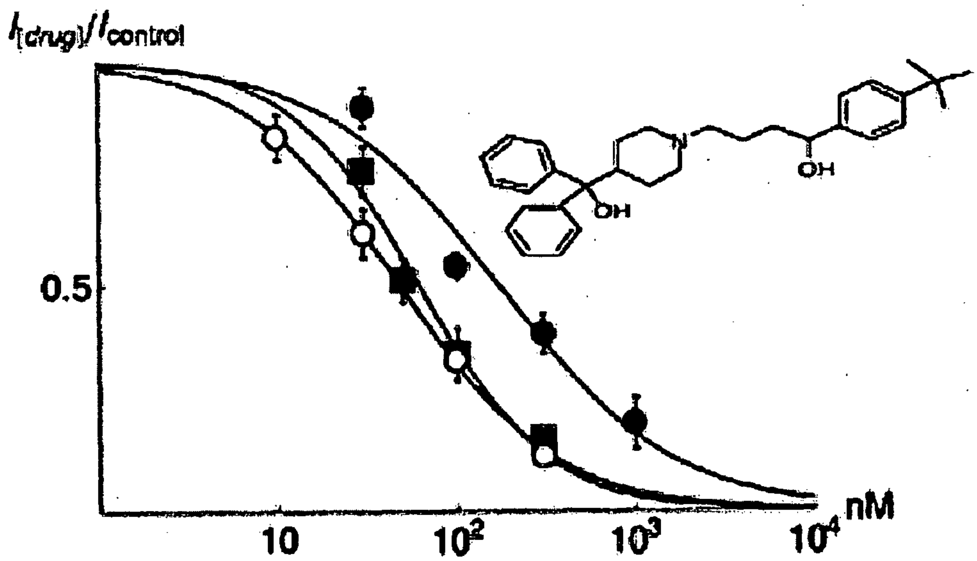

# Figure 4 (cont.)

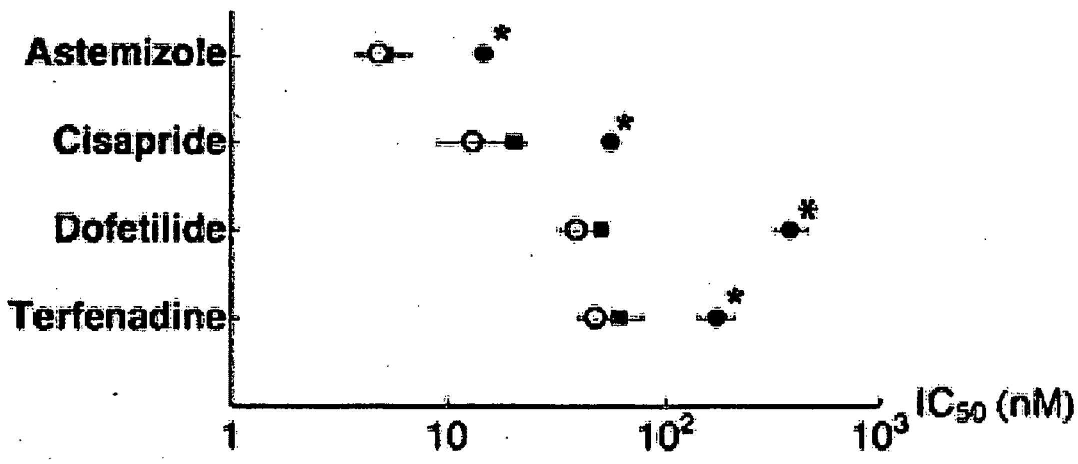

# Figure 5

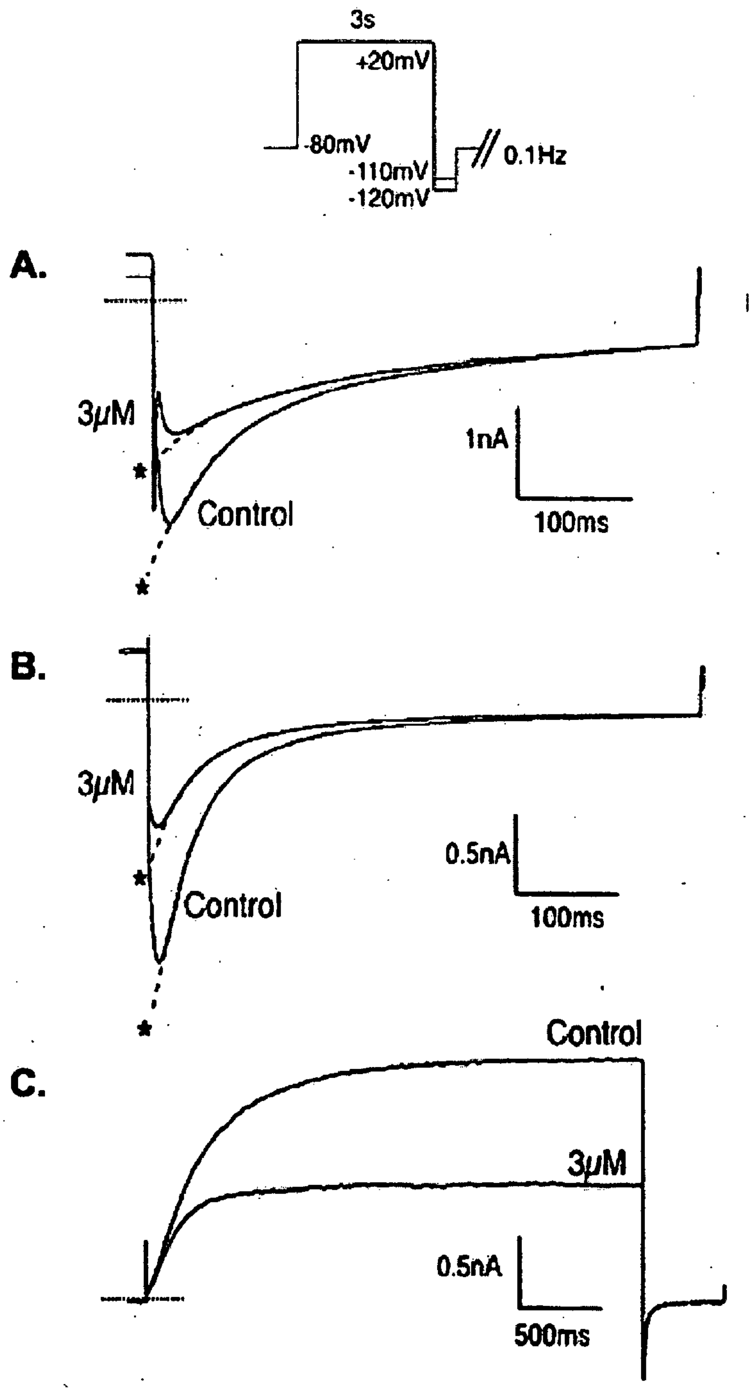
3s
+20mV
-80mV
-110mV
-120mV
0.1Hz
A.
3μM
Control
1nA
100ms
B.
3μM
Control
0.5nA
100ms
C.
Control
3μM
0.5nA
500ms

# Figure 6

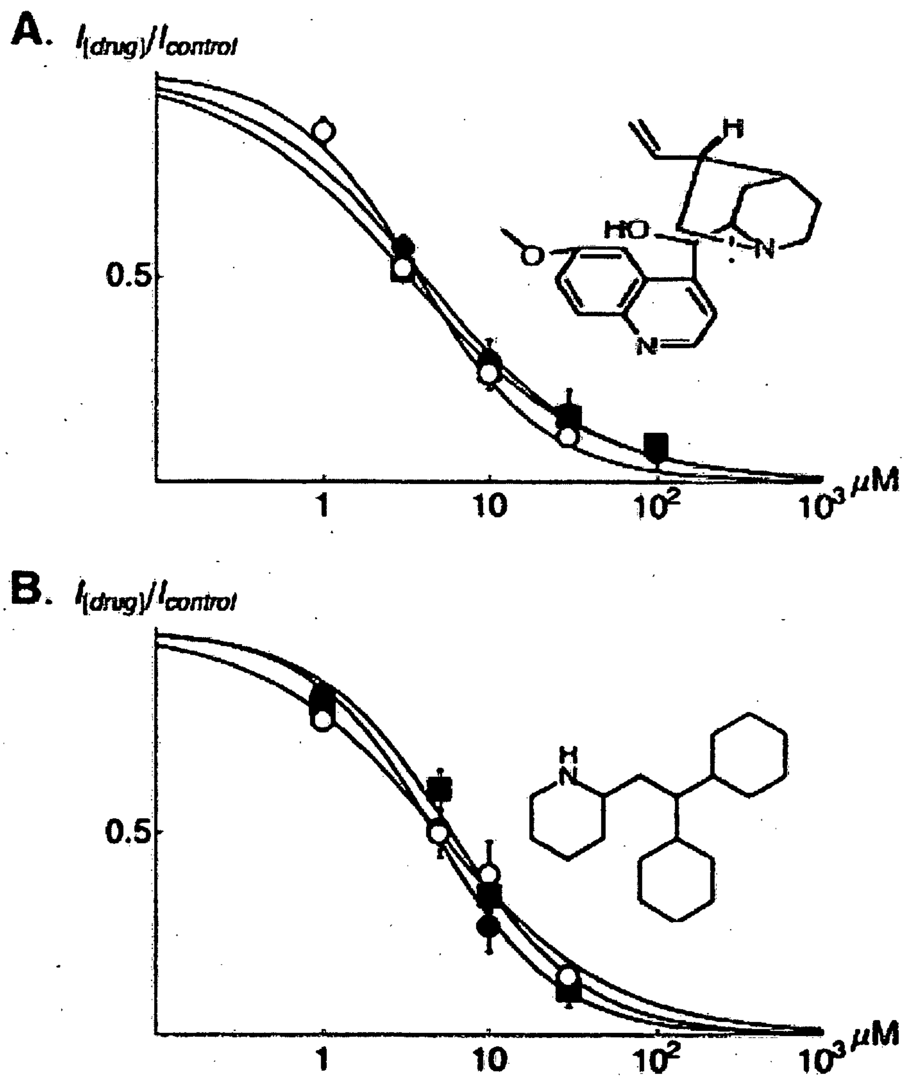
A.
I[drug]/I control
0.5
1
10
10^2
10^3 μM
B.
I[drug]/I control
0.5
1
10
10^2
10^3 μM

# Figure 6 (cont.)

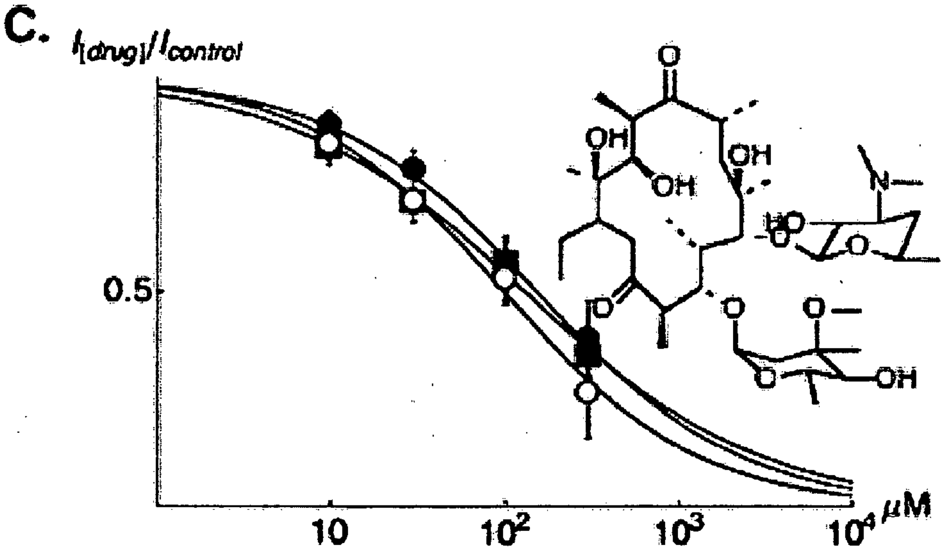


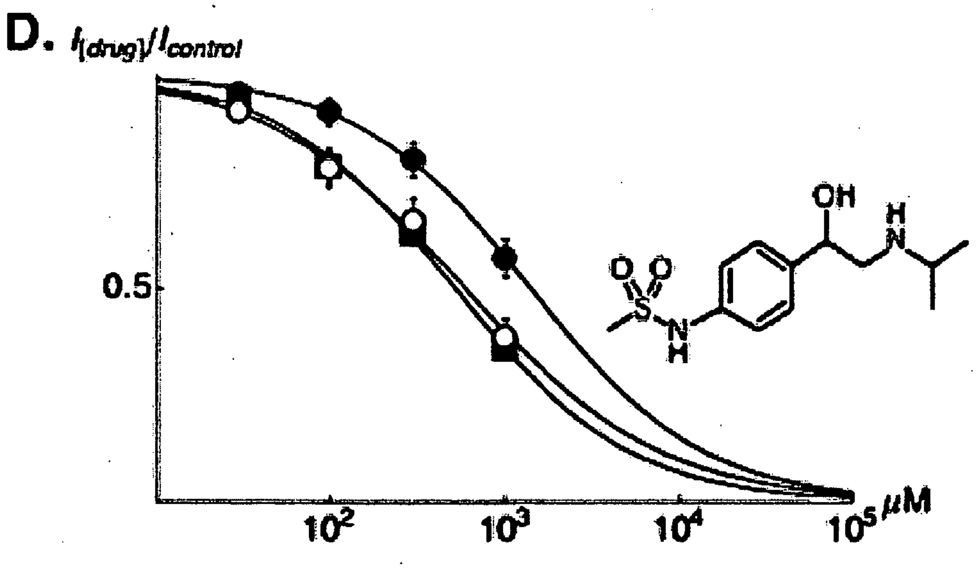

Figure 6 (cont.)

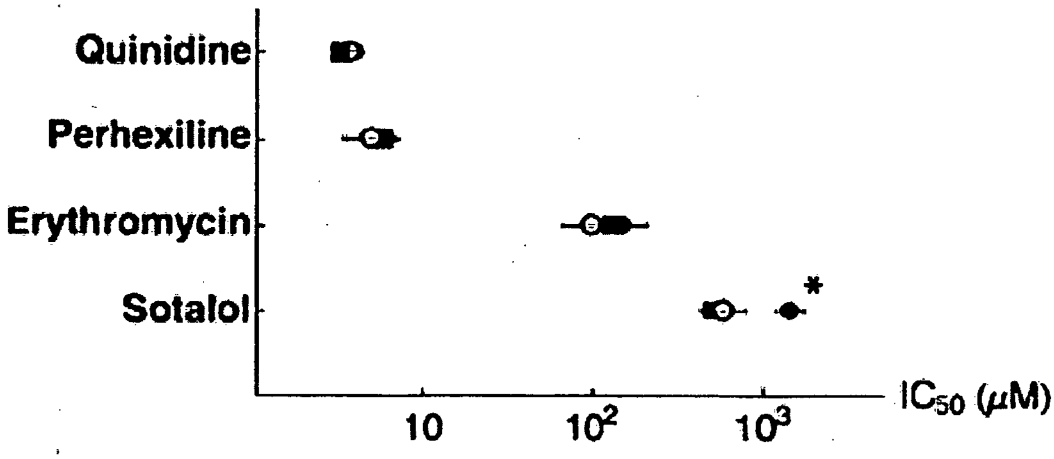

# Figure 7

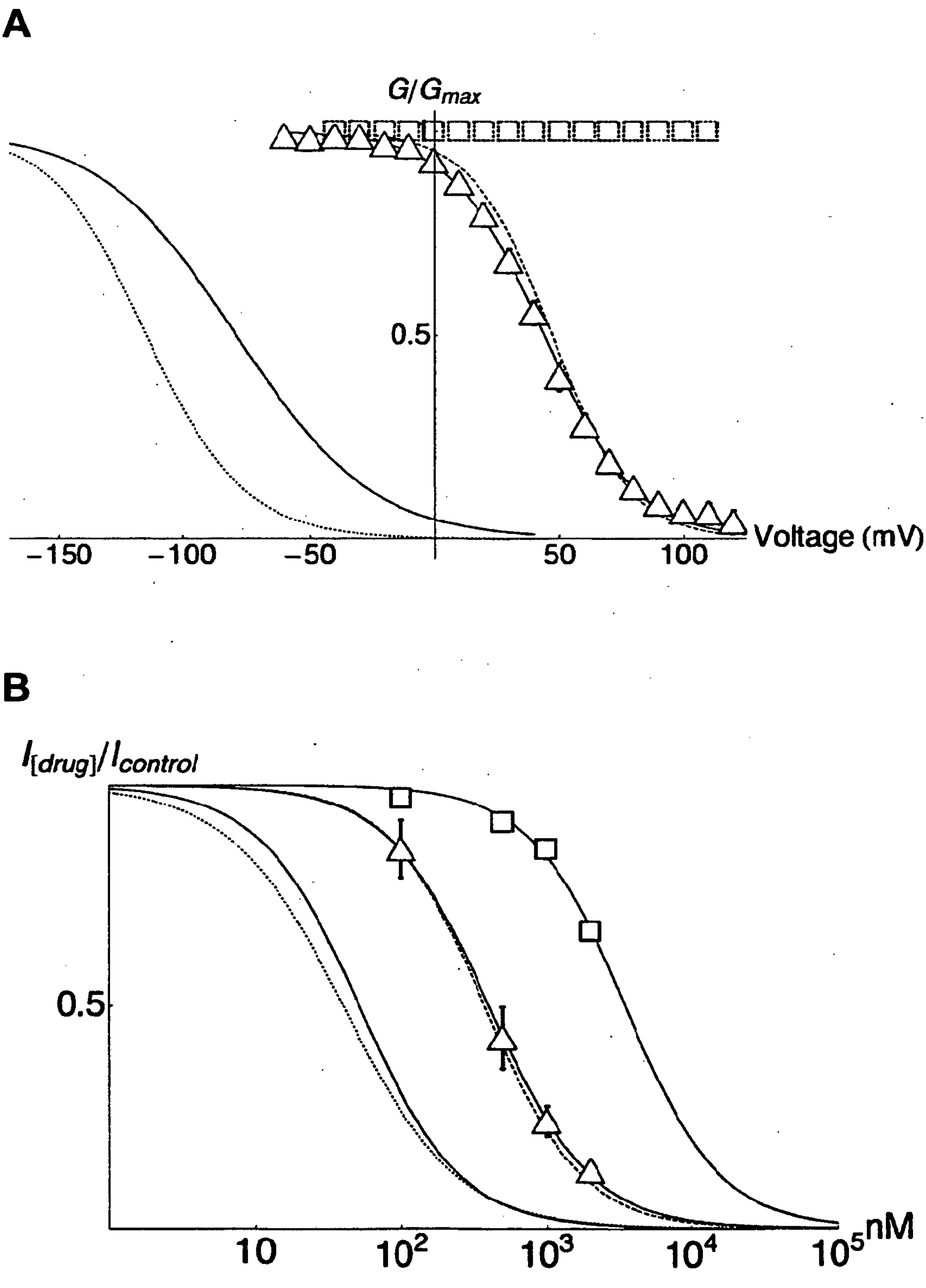
A
G/Gmax
0.5
-150
-100
-50
50
100
Voltage (mV)
B
I[drug]/Icontrol
0.5
10
10^2
10^3
10^4
10^5
nM

# Figure 8

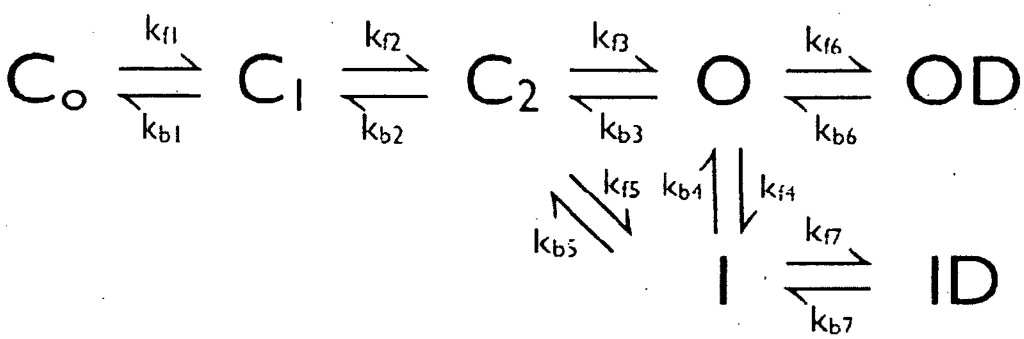


# Figure 9

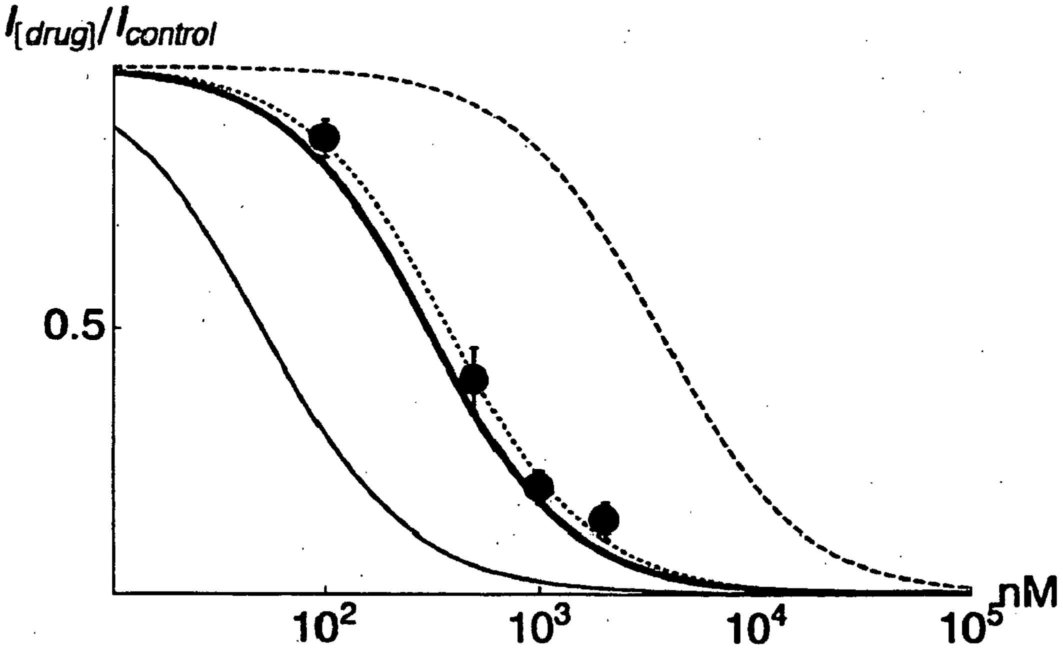

# Figure 10

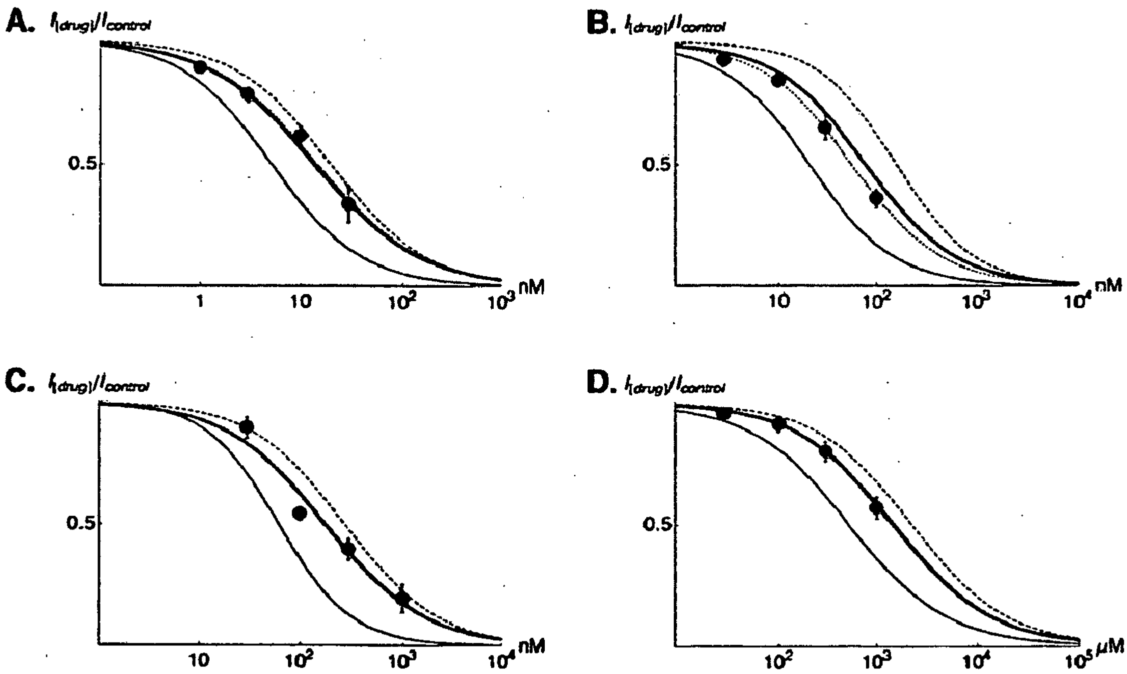
A. $I_{(drug)}/I_{control}$
0.5
1
10
$10^2$
$10^3$
nM
B. $I_{(drug)}/I_{control}$
0.5
10
$10^2$
$10^3$
$10^4$
nM
C. $I_{(drug)}/I_{control}$
0.5
10
$10^2$
$10^3$
$10^4$
nM
D. $I_{(drug)}/I_{control}$
0.5
$10^2$
$10^3$
$10^4$
$10^5$
μM

# Figure 11

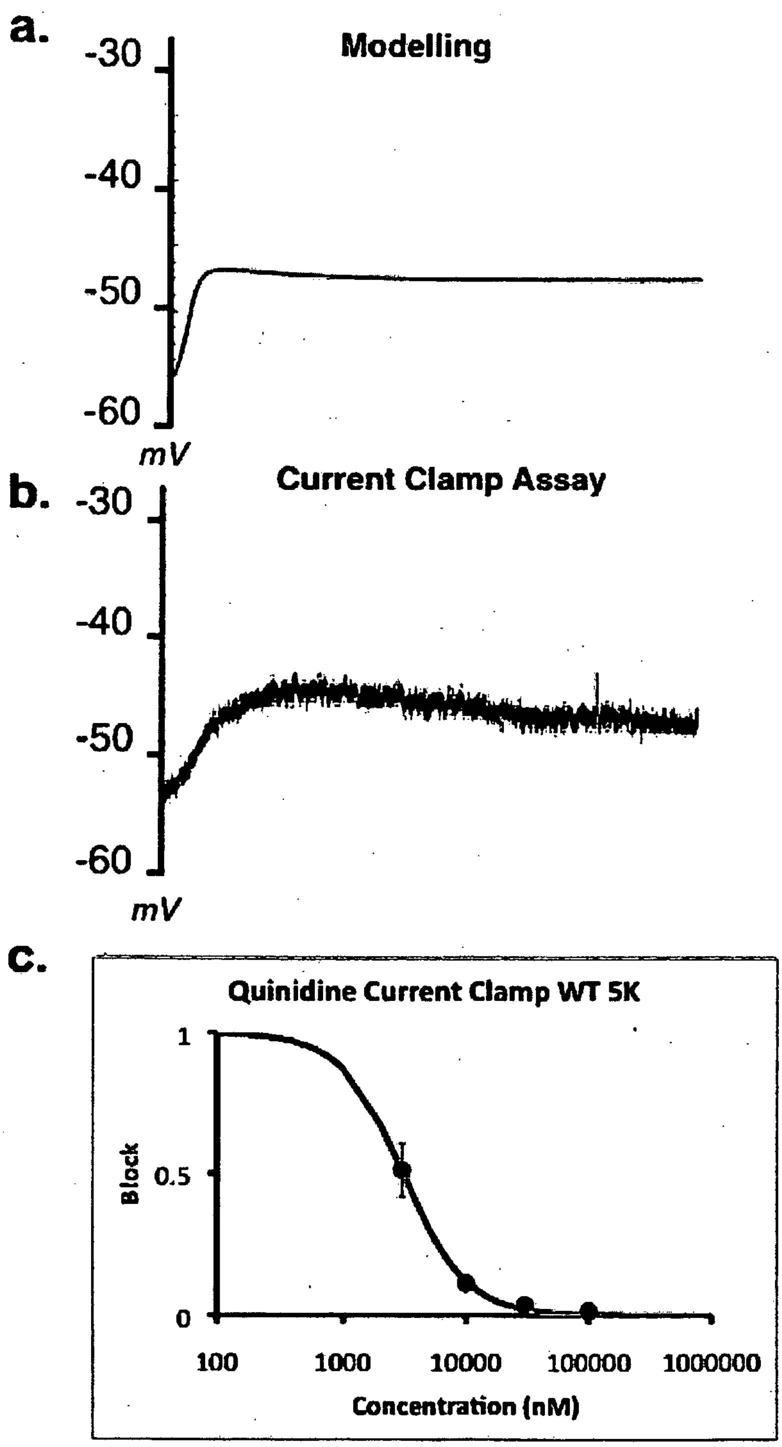
a.
Modelling
-30
-40
-50
-60
mV
b.
Current Clamp Assay
-30
-40
-50
-60
mV
c.
Quinidine Current Clamp WT 5K
Block
1
0.5
0
100
1000
10000
100000
1000000
Concentration (nM)

# Figure 12

ATGCCGGTGCGGAGGGGCCACGTCGCGCCGCAGAACACCTTCCTGGACACCATCATCCGCAAGTTTGAGG
GCCAGAGCCGTAAGTTCATCATCGCCAACGCTCGGGTGGAGAACTGCGCCGTCATCTACTGCAACGACGG
CTTCTGCGAGCTGTGCGGCTACTCGCGGGCCGAGGTGATGCAGCGACCCTGCACCTGCGACTTCCTGCAC
GGGCCGCGCACGCAGCGCCGCGCTGCCGCGCAGATCGCGCAGGCACTGCTGGGCGCCGAGGAGCGCAAAG
TGGAAATCGCCTTCTACCGGAAAGATGGGAGCTGCTTCCTATGTCTGGTGGATGTGGTGCCCGTGAAGAA
CGAGGATGGGGCTGTCATCATGTTCATCCTCAATTTCGAAGTGGTGATGGAGAAGGACATGGTGGGGTCC
CCGGCTCATGACACCAACCACCGGGGCCCCCCCACCAGCTGGCTGGCCCCAGGCCGCGCCAAGACCTTCC
GCCTGAAGCTGCCCGCGCTGCTGGCGCTGACGGCCCGGGAGTCGTCGGTGCGGTCGGGCGGCGCGGGCGG
CGCGGGCGCCCCGGGGGCCGTGGTGGTGGACGTGGACCTGACGCCCGCGGCACCCAGCAGCGAGTCGCTG
GCCCTGGACGAAGTGACAGCCATGGACAACCACGTGGCAGGGCTCGGGCCCGCGGAGGAGCGGCGTGCGC
TGGTGGGTCCCGGCTCTCCGCCCCGCTCAGCGCCCGGCCAGCTCCCATCGCCCCGGGCGCACAGCCTCAA
CCCCGACGCCTCGGGCTCCAGCTGCAGCCTGGCCCGGACGCGCTCCCGAGAAAGCTGCGCCAGCGTGCGC
CGCGCCTCGTCGGCCGACGACATCGAGGCCATGCGCGCCGGGGTGCTGCCCCCGCCACCGCGCCACGCCA
GCACCGGGGCCATGCACCCACTGCGCAGCGGCTTGCTCAACTCCACCTCGGACTCCGACCTCGTGCGCTA
CCGCACCATTAGCAAGATTCCCCAAATCACCCTCAACTTTGTGGACCTCAAGGGCGACCCCTTCTTGGCT
TCGCCCACCAGTGACCGTGAGATCATAGCACCTAAGATAAAGGAGCGAACCCACAATGTCACTGAGAAGG
TCACCCAGGTCCTGTCCCTGGGCGCCGACGTGCTGCCTGAGTACAAGCTGCAGGCACCGCGCATCCACCG
CTGGACCATCCTGCATTACAGCCCCTTCAAGGCCGTGTGGGACTGGCTCATCCTGCTGCTGGTCATCTAC
ACGGCTGTCTTCACACCCTACTCGGCTGCCTTCCTGCTTAAGGAGACGGAAGAAGGCCCGCCGGCGACCG
AGTGTGGCTATGCATGCCAGCCGCTGGCTGTGGTGGACCTCATCGTGGACATCATGTTCATTGTGGACAT
CCTCATCAACTTCCGCACCACCTACGTCAATGCCAACGAGGAGGTGGTCAGCCACCCCGGCCGCATCGCC
GTCCACTACTTCAAGGGCTGGTTCCTCATCGACATGGTGGCCGCCATCCCCTTCGACCTGCTCATCTTCG
GCTCTGGCTCTGAGGAGCTGATCGGGCTG**YKN**AAGACGGCGCGCCTGCTGCGG**YYN**GTGCGC**GYNGYN**CG
G**MAR**CTGGATCGCTACTCAGAGTACGGCGCGGCCGTGCTGTTCTTGCTCATGTGTACCTTTGCGCTCATC
GCGCACTGGCTAGCCTGCATCTGGTACGCAATTGGCAACATGGAGCAGCCACACATGGACTCACGCATC**D**
**SN**TGGCTG**MANRAY**CTGGGCGACCAGATAGGCAAACCCTACAACAGCTCAGGCCTGGGCGGCCCCTCCAT
CAAGGACAAGTATGTGACGGCGCTCTACTTCACCTTCAGCAGCCTCACCAGTGTGGGCTTCGGCAACGTC
TCTCCCAACACCAACTCAGAGAAGATCTTCTCCATCTGCGTCATGCTCATTGGCTCCCTCATGTATGCTA
GCATCTTCGGCAACGTGTCGGCCATCATCCAGCGGCTGTACTCGGGCACAGCCCGCTACCACACACAGAT
GCTGCGGGTGCGGGAGTTCATCCGCTTCCACCAGATCCCCAATCCCCTGCGCCAGCGCCTCGAGGAGTAC
TTCCAGCACGCCTGGTCCTACACCAACGGCATCGACATGAACGCGGTGCTGAAGGGCTTCCCTGAGTGCC
TGCAGGCTGACATCTGCCTGCACCTGAACCGGTCACTGCTGCAGCACTGCAAACCCTTCCGAGGGGCCAC
CAAGGGCTGCCTTCGGGCCCTGGCCATGAAGTTCAAGACCACACATGCACCGCCAGGGGACACACTGGTG
CATGCTGGGGACCTGCTCACCGCCCTGTACTTCATCTCCCGGGGCTCCATCGAGATCCTGCGGGGCGACG
TCGTCGTGGCCATCCTGGGGAAGAATGACATCTTTGGGGAGCCTCTGAACCTGTATGCAAGGCCTGGCAA
GTCGAACGGGGATGTGCGGGCCCTCACCTACTGTGACCTACACAAGATCCATCGGGACGACCTGCTGGAG
GTGCTGGACATGTACCCTGAGTTCTCTGATCACTTCTGGTCCAGCCTGGAGATCACCTTCAACCTGCGAG
ATACCAACATGATCCCGGGCTCCCCCGGCAGTACGGAGTTAGAGGGTGGCTTCAGTCGGCAACGCAAGCG
CAAGTTGTCCTTCCGCAGGCGCACGGACAAGGACACGGAGCAGCCAGGGGAGGTGTCGGCCTTGGGGCCG
GGCCGGGCGGGGGCAGGGCCGAGTAGCCGGGGCCGGCCGGGGGGGCCGTGGGGGAGAGCCCGTCCAGTG
GCCCCTCCAGCCCTGAGAGCAGTGAGGATGAGGGCCCAGGCCGCAGCTCCAGCCCCCTCCGCCTGGTGCC
CTTCTCCAGCCCCAGGCCCCCCGGAGAGCCGCCGGGTGGGGAGCCCCTGATGGAGGACTGCGAGAAGAGC
AGCGACACTTGCAACCCCCTGTCAGGCGCCTTCTCAGGAGTGTCCAACATTTTCAGCTTCTGGGGGGACA
GTCGGGGCCGCCAGTACCAGGAGCTCCCTCGATGCCCCGCCCCCACCCCCAGCCTCCTCAACATCCCCCT
CTCCAGCCCGGGTCGGCGGCCCCGGGGCGACGTGGAGAGCAGGCTGGATGCCCTCCAGCGCCAGCTCAAC
AGGCTGGAGACCCGGCTGAGTGCAGACATGGCCACTGTCCTGCAGCTGCTACAGAGGCAGATGACGCTGG
TCCCGCCCGCCTACAGTGCTGTGACCACCCCGGGGCCTGGCCCCACTTCCACATCCCCGCTGTTGCCCGT
CAGCCCCCTCCCCACCCTCACCTTGGACTCGCTTTCTCAGGTTTCCCAGTTCATGGCGTGTGAGGAGCTG

CCCCCGGGGGCCCCAGAGCTTCCCCAAGAAGGCCCCACACGACGCCTCTCCCTACCGGGCCAGCTGGGGG
CCCTCACCTCCCAGCCCCTGCACAGACACGGCTCGGACCCGGGCAGTACGCGTTAG

# Figure 13

```
synth   ATGCCGGTGCGGAGGGGCCACGTCGCGCCGCAGAACACCTTCCTGGACACCATCATCCGC 60
WT      ATGCCGGTGCGGAGGGGCCACGTCGCGCCGCAGAACACCTTCCTGGACACCATCATCCGC 60

synth   AAGTTTGAGGGCCAGAGCCGTAAGTTCATCATCGCCAACGCTCGGGTGGAGAACTGCGCC 120
WT      AAGTTTGAGGGCCAGAGCCGTAAGTTCATCATCGCCAACGCTCGGGTGGAGAACTGCGCC 120

synth   GTCATCTACTGCAACGACGGCTTCTGCGAGCTGTGCGGCTACTCGCGGGCCGAGGTGATG 180
WT      GTCATCTACTGCAACGACGGCTTCTGCGAGCTGTGCGGCTACTCGCGGGCCGAGGTGATG 180

synth   CAGCGACCCTGCACCTGCGACTTCCTGCACGGGCCGCGCACGCAGCGCCGCGCTGCCGCG 240
WT      CAGCGACCCTGCACCTGCGACTTCCTGCACGGGCCGCGCACGCAGCGCCGCGCTGCCGCG 240

synth   CAGATCGCGCAGGCACTGCTGGGCGCCGAGGAGCGCAAAGTGGAAATCGCCTTCTACCGG 300
WT      CAGATCGCGCAGGCACTGCTGGGCGCCGAGGAGCGCAAAGTGGAAATCGCCTTCTACCGG 300

synth   AAAGATGGGAGCTGCTTCCTATGTCTGGTGGATGTGGTGCCCGTGAAGAACGAGGATGGG 360
WT      AAAGATGGGAGCTGCTTCCTATGTCTGGTGGATGTGGTGCCCGTGAAGAACGAGGATGGG 360

synth   GCTGTCATCATGTTCATCCTCAATTTCGAAGTGGTGATGGAGAAGGACATGGTGGGGTCC 420
WT      GCTGTCATCATGTTCATCCTCAATTTCGAGGTGGTGATGGAGAAGGACATGGTGGGGTCC 420

synth   CCGGCTCATGACACCAACCACCGGGGCCCCCCCACCAGCTGGCTGGCCCCAGGCCGCGCC 480
WT      CCGGCTCATGACACCAACCACCGGGGCCCCCCCACCAGCTGGCTGGCCCCAGGCCGCGCC 480

synth   AAGACCTTCCGCCTGAAGCTGCCCGCGCTGCTGGCGCTGACGGCCCGGGAGTCGTCGGTG 540
WT      AAGACCTTCCGCCTGAAGCTGCCCGCGCTGCTGGCGCTGACGGCCCGGGAGTCGTCGGTG 540

synth   CGGTCGGGCGGCGCGGGCGGCGCGGGCGCCCCGGGGGCCGTGGTGGTGGACGTGGACCTG 600
WT      CGGTCGGGCGGCGCGGGCGGCGCGGGCGCCCCGGGGGCCGTGGTGGTGGACGTGGACCTG 600

synth   ACGCCCGCGGCACCCAGCAGCGAGTCGCTGGCCCTGGACGAAGTGACAGCCATGGACAAC 660
WT      ACGCCCGCGGCACCCAGCAGCGAGTCGCTGGCCCTGGACGAAGTGACAGCCATGGACAAC 660

synth   CACGTGGCAGGGCTCGGGCCCGCGGAGGAGCGGCGTGCGCTGGTGGGTCCCGGCTCTCCG 720
WT      CACGTGGCAGGGCTCGGGCCCGCGGAGGAGCGGCGTGCGCTGGTGGGTCCCGGCTCTCCG 720

synth   CCCCGCTCAGCGCCCGGCCAGCTCCCATCGCCCCGGGCGCACAGCCTCAACCCCGACGCC 780
WT      CCCCGCAGCGCGCCCGGCCAGCTCCCATCGCCCCGGGCGCACAGCCTCAACCCCGACGCC 780

synth   TCGGGCTCCAGCTGCAGCCTGGCCCGGACGCGCTCCCGAGAAAGCTGCGCCAGCGTGCGC 840
WT      TCGGGCTCCAGCTGCAGCCTGGCCCGGACGCGCTCCCGAGAAAGCTGCGCCAGCGTGCGC 840

synth   CGCGCCTCGTCGGCCGACGACATCGAGGCCATGCGCGCCGGGGTGCTGCCCCCGCCACCG 900
WT      CGCGCCTCGTCGGCCGACGACATCGAGGCCATGCGCGCCGGGGTGCTGCCCCCGCCACCG 900

synth   CGCCACGCCAGCACCGGGGCCATGCACCCACTGCGCAGCGGCTTGCTCAACTCCACCTCG 960
WT      CGCCACGCCAGCACCGGGGCCATGCACCCACTGCGCAGCGGCTTGCTCAACTCCACCTCG 960

synth   GACTCCGACCTCGTGCGCTACCGCACCATTAGCAAGATTCCCCAAATCACCCTCAACTTT 1020
WT      GACTCCGACCTCGTGCGCTACCGCACCATTAGCAAGATTCCCCAAATCACCCTCAACTTT 1020

synth   GTGGACCTCAAGGGCGACCCCTTCTTGGCTTCGCCCACCAGTGACCGTGAGATCATAGCA 1080
WT      GTGGACCTCAAGGGCGACCCCTTCTTGGCTTCGCCCACCAGTGACCGTGAGATCATAGCA 1080

synth   CCTAAGATAAAGGAGCGAACCCACAATGTCACTGAGAAGGTCACCCAGGTCCTGTCCCTG 1140
WT      CCTAAGATAAAGGAGCGAACCCACAATGTCACTGAGAAGGTCACCCAGGTCCTGTCCCTG 1140
```

```
synth  GGCGCCGACGTGCTGCCTGAGTACAAGCTGCAGGCACCGCGCATCCACCGCTGGACCATC 1200
WT     GGCGCCGACGTGCTGCCTGAGTACAAGCTGCAGGCACCGCGCATCCACCGCTGGACCATC 1200

synth  CTGCATTACAGCCCCTTCAAGGCCGTGTGGGACTGGCTCATCCTGCTGCTGGTCATCTAC 1260
WT     CTGCATTACAGCCCCTTCAAGGCCGTGTGGGACTGGCTCATCCTGCTGCTGGTCATCTAC 1260

synth  ACGGCTGTCTTCACACCCTACTCGGCTGCCTTCCTGCTTAAGGAGACGGAAGAAGGCCCG 1320
WT     ACGGCTGTCTTCACACCCTACTCGGCTGCCTTCCTGCTGAAGGAGACGGAAGAAGGCCCG 1320

synth  CCGGCGACCGAGTGTGGCTATGCATGCCAGCCGCTGGCTGTGGTGGACCTCATCGTGGAC 1380
WT     CCTGCTACCGAGTGTGGCTACGCCTGCCAGCCGCTGGCTGTGGTGGACCTCATCGTGGAC 1380

synth  ATCATGTTCATTGTGGACATCCTCATCAACTTCCGCACCACCTACGTCAATGCCAACGAG 1440
WT     ATCATGTTCATTGTGGACATCCTCATCAACTTCCGCACCACCTACGTCAATGCCAACGAG 1440

synth  GAGGTGGTCAGCCACCCCGGCCGCATCGCCGTCCACTACTTCAAGGGCTGGTTCCTCATC 1500
WT     GAGGTGGTCAGCCACCCCGGCCGCATCGCCGTCCACTACTTCAAGGGCTGGTTCCTCATC 1500

synth  GACATGGTGGCCGCCATCCCCTTCGACCTGCTCATCTTCGGCTCTGGCTCTGAGGAGCTG 1560
WT     GACATGGTGGCCGCCATCCCCTTCGACCTGCTCATCTTCGGCTCTGGCTCTGAGGAGCTG 1560

synth  ATCGGGCTGYKNAAGACGGCGCGCCTGCTGCGGYYNGTGCGCGYNGYNCGGMARCTGGAT 1620
WT     ATCGGGCTGCTGAAGACTGCGCGGCTGCTGCGGCTGGTGCGCGTGGCGCGGAAGCTGGAT 1620

synth  CGCTACTCAGAGTACGGCGCGGCCGTGCTGTTCTTGCTCATGTGTACCTTTGCGCTCATC 1680
WT     CGCTACTCAGAGTACGGCGCGGCCGTGCTGTTCTTGCTCATGTGCACCTTTGCGCTCATC 1680

synth  GCGCACTGGCTAGCCTGCATCTGGTACGCAATTGGCAACATGGAGCAGCCACACATGGAC 1740
WT     GCGCACTGGCTAGCCTGCATCTGGTACGCCATCGGCAACATGGAGCAGCCACACATGGAC 1740

synth  TCACGCATCDSNTGGCTGMANRAYCTGGGCGACCAGATAGGCAAACCCTACAACAGCTCA 1800
WT     TCACGCATCGGCTGGCTGCACAACCTGGGCGACCAGATAGGCAAACCCTACAACAGCAGC 1800

synth  GGCCTGGGCGGCCCCTCCATCAAGGACAAGTATGTGACGGCGCTCTACTTCACCTTCAGC 1860
WT     GGCCTGGGCGGCCCCTCCATCAAGGACAAGTATGTGACGGCGCTCTACTTCACCTTCAGC 1860

synth  AGCCTCACCAGTGTGGGCTTCGGCAACGTCTCTCCCAACACCAACTCAGAGAAGATCTTC 1920
WT     AGCCTCACCAGTGTGGGCTTCGGCAACGTCTCTCCCAACACCAACTCAGAGAAGATCTTC 1920

synth  TCCATCTGCGTCATGCTCATTGGCTCCCTCATGTATGCTAGCATCTTCGGCAACGTGTCG 1980
WT     TCCATCTGCGTCATGCTCATTGGCTCCCTCATGTATGCTAGCATCTTCGGCAACGTGTCG 1980

synth  GCCATCATCCAGCGGCTGTACTCGGGCACAGCCCGCTACCACACACAGATGCTGCGGGTG 2040
WT     GCCATCATCCAGCGGCTGTACTCGGGCACAGCCCGCTACCACACACAGATGCTGCGGGTG 2040

synth  CGGGAGTTCATCCGCTTCCACCAGATCCCCAATCCCCTGCGCCAGCGCCTCGAGGAGTAC 2100
WT     CGGGAGTTCATCCGCTTCCACCAGATCCCCAATCCCCTGCGCCAGCGCCTCGAGGAGTAC 2100

synth  TTCCAGCACGCCTGGTCCTACACCAACGGCATCGACATGAACGCGGTGCTGAAGGGCTTC 2160
WT     TTCCAGCACGCCTGGTCCTACACCAACGGCATCGACATGAACGCGGTGCTGAAGGGCTTC 2160

synth  CCTGAGTGCCTGCAGGCTGACATCTGCCTGCACCTGAACCGGTCACTGCTGCAGCACTGC 2220
WT     CCTGAGTGCCTGCAGGCTGACATCTGCCTGCACCTGAACCGCTCACTGCTGCAGCACTGC 2220

synth  AAACCCTTCCGAGGGGCCACCAAGGGCTGCCTTCGGGCCCTGGCCATGAAGTTCAAGACC 2280
WT     AAACCCTTCCGAGGGGCCACCAAGGGCTGCCTTCGGGCCCTGGCCATGAAGTTCAAGACC 2280

synth  ACACATGCACCGCCAGGGGACACACTGGTGCATGCTGGGGACCTGCTCACCGCCCTGTAC 2340
WT     ACACATGCACCGCCAGGGGACACACTGGTGCATGCTGGGGACCTGCTCACCGCCCTGTAC 2340
```

```
synth  TTCATCTCCCGGGGCTCCATCGAGATCCTGCGGGGCGACGTCGTCGTGGCCATCCTGGGG 2400
WT     TTCATCTCCCGGGGCTCCATCGAGATCCTGCGGGGCGACGTCGTCGTGGCCATCCTGGGG 2400

synth  AAGAATGACATCTTTGGGGAGCCTCTGAACCTGTATGCAAGGCCTGGCAAGTCGAACGGG 2460
WT     AAGAATGACATCTTTGGGGAGCCTCTGAACCTGTATGCAAGGCCTGGCAAGTCGAACGGG 2460

synth  GATGTGCGGGCCCTCACCTACTGTGACCTACACAAGATCCATCGGGACGACCTGCTGGAG 2520
WT     GATGTGCGGGCCCTCACCTACTGTGACCTACACAAGATCCATCGGGACGACCTGCTGGAG 2520

synth  GTGCTGGACATGTACCCTGAGTTCTCTGATCACTTCTGGTCCAGCCTGGAGATCACCTTC 2580
WT     GTGCTGGACATGTACCCTGAGTTCTCCGACCACTTCTGGTCCAGCCTGGAGATCACCTTC 2580

synth  AACCTGCGAGATACCAACATGATCCCGGGCTCCCCCGGCAGTACGGAGTTAGAGGGTGGC 2640
WT     AACCTGCGAGATACCAACATGATCCCGGGCTCCCCCGGCAGTACGGAGTTAGAGGGTGGC 2640

synth  TTCAGTCGGCAACGCAAGCGCAAGTTGTCCTTCCGCAGGCGCACGGACAAGGACACGGAG 2700
WT     TTCAGTCGGCAACGCAAGCGCAAGTTGTCCTTCCGCAGGCGCACGGACAAGGACACGGAG 2700

synth  CAGCCAGGGGAGGTGTCGGCCTTGGGGCCGGGCCGGGCGGGGGCAGGGCCGAGTAGCCGG 2760
WT     CAGCCAGGGGAGGTGTCGGCCTTGGGGCCGGGCCGGGCGGGGGCAGGGCCGAGTAGCCGG 2760

synth  GGCCGGCCGGGGGGGCCGTGGGGGGAGAGCCCGTCCAGTGGCCCCTCCAGCCCTGAGAGC 2820
WT     GGCCGGCCGGGGGGGCCGTGGGGGGAGAGCCCGTCCAGTGGCCCCTCCAGCCCTGAGAGC 2820

synth  AGTGAGGATGAGGGCCCAGGCCGCAGCTCCAGCCCCCTCCGCCTGGTGCCCTTCTCCAGC 2880
WT     AGTGAGGATGAGGGCCCAGGCCGCAGCTCCAGCCCCCTCCGCCTGGTGCCCTTCTCCAGC 2880

synth  CCCAGGCCCCCCGGAGAGCCGCCGGGTGGGGAGCCCCTGATGGAGGACTGCGAGAAGAGC 2940
WT     CCCAGGCCCCCCGGAGAGCCGCCGGGTGGGGAGCCCCTGATGGAGGACTGCGAGAAGAGC 2940

synth  AGCGACACTTGCAACCCCCTGTCAGGCGCCTTCTCAGGAGTGTCCAACATTTTCAGCTTC 3000
WT     AGCGACACTTGCAACCCCCTGTCAGGCGCCTTCTCAGGAGTGTCCAACATTTTCAGCTTC 3000

synth  TGGGGGGACAGTCGGGGCCGCCAGTACCAGGAGCTCCCTCGATGCCCCGCCCCCACCCCC 3060
WT     TGGGGGGACAGTCGGGGCCGCCAGTACCAGGAGCTCCCTCGATGCCCCGCCCCCACCCCC 3060

synth  AGCCTCCTCAACATCCCCCTCTCCAGCCCGGGTCGGCGGCCCCGGGGCGACGTGGAGAGC 3120
WT     AGCCTCCTCAACATCCCCCTCTCCAGCCCGGGTCGGCGGCCCCGGGGCGACGTGGAGAGC 3120

synth  AGGCTGGATGCCCTCCAGCGCCAGCTCAACAGGCTGGAGACCCGGCTGAGTGCAGACATG 3180
WT     AGGCTGGATGCCCTCCAGCGCCAGCTCAACAGGCTGGAGACCCGGCTGAGTGCAGACATG 3180

synth  GCCACTGTCCTGCAGCTGCTACAGAGGCAGATGACGCTGGTCCCGCCCGCCTACAGTGCT 3240
WT     GCCACTGTCCTGCAGCTGCTACAGAGGCAGATGACGCTGGTCCCGCCCGCCTACAGTGCT 3240

synth  GTGACCACCCCGGGGCCTGGCCCCACTTCCACATCCCCGCTGTTGCCCGTCAGCCCCCTC 3300
WT     GTGACCACCCCGGGGCCTGGCCCCACTTCCACATCCCCGCTGTTGCCCGTCAGCCCCCTC 3300

synth  CCCACCCTCACCTTGGACTCGCTTTCTCAGGTTTCCCAGTTCATGGCGTGTGAGGAGCTG 3360
WT     CCCACCCTCACCTTGGACTCGCTTTCTCAGGTTTCCCAGTTCATGGCGTGTGAGGAGCTG 3360

synth  CCCCCGGGGGCCCCAGAGCTTCCCCAAGAAGGCCCCACACGACGCCTCTCCCTACCGGGC 3420
WT     CCCCCGGGGGCCCCAGAGCTTCCCCAAGAAGGCCCCACACGACGCCTCTCCCTACCGGGC 3420

synth  CAGCTGGGGGCCCTCACCTCCCAGCCCCTGCACAGACACGGCTCGGACCCGGGCAGTACG 3480
WT     CAGCTGGGGGCCCTCACCTCCCAGCCCCTGCACAGACACGGCTCGGACCCGGGCAGT--- 3477

synth  CGTTAG 3486
WT     ---TAG 3480
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **WALKER BD et al.** *Br J Pharmacol,* 1999, vol. 128 (2), 444-450
- **PERRIN MJ et al.** *Mol Pharmacol.,* November 2008, vol. 74 (5), 1443-52
- **REZAZADEH S et al.** *J Biomol Screen.,* October 2004, vol. 9 (7), 588-97
- **DORN A et al.** *J Biomol Screen.,* June 2005, vol. 10 (4), 339-47
- **LU et al.** *J Physiol.,* 15 December 2001, vol. 537, 843-51